# EUROPEAN PATENT APPLICATION

(11) **EP 4 306 102 A1**
(43) Date of publication of application: **17.01.2024**
(21) Application number: 22766643.5
(22) Date of filing: 25.01.2022
(51) Int. Cl.: A61K 8/891, A61Q 1/02, A61Q 1/04, A61Q 1/10, A61Q 1/12, A61Q 5/00, A61Q 5/12, A61Q 17/04, A61Q 19/00, C08G 77/50, C08L 83/14, C08L 91/00

(54) **COSMETIC PREPARATION**

(30) Priority: 08.03.2021 JP 2021036091
(71) Applicant: SHIN-ETSU CHEMICAL CO., LTD., Tokyo 1000005 (JP)
(72) Inventor: AKABANE Emi, Annaka-shi, Gunma 379-0224 (JP); KONISHI Masayuki, Tokyo 100-0005 (JP)
(74) Representative: Sonnenhauser, Thomas Martin
(86) International application number: PCT/JP2022/002495
(87) International publication number: WO 2022/190676

(57) **Abstract**

The present invention is a cosmetic including the following component (A), the component (A): a hydrosilylated reaction product of an organohydrogen (poly)siloxane (A1) and an organo(poly)siloxane (A2), the hydrosilylated reaction product being an aromaticgroup-modified crosslinked organosiloxane, and a ratio between a total number of moles of aryl groups and aralkyl groups and a number of moles of silicon atoms is 0.34 or more. This enables a highly polar oil and an ultraviolet-ray absorbent to be stably blended, and provides a cosmetic having good feeling and feel of use.

## Description

### TECHNICAL FIELD

The present invention relates to a cosmetic using an aromatic-group-modified crosslinked organosiloxane.

### BACKGROUND ART

As a method for stably blending a silicone oil, particularly a dimethylsilicone oil, into a cosmetic or a personal care composition, an organosiloxane polymer having a three-dimensional crosslinked structure is proposed.

For example, Patent Documents 1 and 2 propose an elastomer composition containing a crosslinked elastomer composed of a hydrogenpolysiloxane and a diene compound, and a diluting agent. The diene compound represented by 1,4-pentadiene and 1,5-hexadiene has uncomfortable characteristic odor, and there is a problem of unsuitableness for cosmetic use if the diene compound remains. A paste composition obtained by mixing a specific silicone polymer and a low-viscosity silicone oil under shearing, proposed in Patent Document 3, can be blended into a cosmetic to provide not only temporal stability but also feel of use and usability having smooth and plain feeling. However, it is difficult to mention that the effect is sufficiently satisfactory with oil agents other than the silicone oil, for example, a hydrocarbon oil, an ester oil, and a highly polar oil containing an ultraviolet-ray absorbent.

To solve this problem, proposed is use of a crosslinked organosiloxane polymer having high compatibility with a silicone oil, a hydrocarbon oil, and an ester oil by introducing a long-chain alkyl group (Patent Document 4). However, the effect is unsatisfactory on the compatibility with a highly polar oil containing an ultraviolet-ray absorbent.

Meanwhile, a silicone oil having a phenyl group is known to have compatibility with the ultraviolet-ray absorbent, and a crosslinked organosiloxane having a phenyl group is expected to have compatibility with the ultraviolet-ray absorbent. The aforementioned documents describe use of the phenyl group as a substituent that can be bonded to a silicon atom. Use examples of the crosslinked organosiloxane having a phenyl group for a cosmetic include Patent Document 5, which uses unique feeling imparted by introducing the phenyl group, and Patent Document 6, which has a purpose of stable blending of a phenyl-modified silicone oil. The usefulness of the phenyl group is acknowledged at a certain degree, but the compatibility with the ultraviolet-ray absorbent is not disclosed. Such an organosiloxane may be equally treated as a crosslinked organosiloxane polymer having no phenyl group nor compatibility with the ultraviolet-ray absorbent in some cases, as Patent Documents 7, 8, etc., and performance and structure as a cosmetic raw material remain of a room for improvement.

### CITATION LIST

### PATENT LITERATURE

Patent Document 1: US 5654362 B
Patent Document 2: JP 2005-537364 A
Patent Document 3: JP H1-207354 A
Patent Document 4: WO 2003/024413 A1
Patent Document 5: JP 2013-103885 A
Patent Document 6: JP 2011-231102 A
Patent Document 7: JP 2015-086196 A
Patent Document 8: JP 2020-029409 A

### SUMMARY OF INVENTION

### TECHNICAL PROBLEM

The present invention has been made in view of the above circumstances. An object of the present invention is to provide a cosmetic in which a highly polar oil and an ultraviolet-ray absorbent can be stably blended and that has good feeling, feel of use, and coatability.

### SOLUTION TO PROBLEM

To solve the above problem, the present invention provides a cosmetic comprising the following component (A),
the component (A): a hydrosilylated reaction product of the following (A1) and (A2), the hydrosilylated reaction product being an aromatic-group-modified crosslinked organosiloxane, and a ratio between a total number of moles of aryl groups and aralkyl groups and a number of moles of silicon atoms is 0.34 or more,
(A1) an organohydrogen (poly)siloxane represented by the following general formula (1) and having two or more silicon-atom-bonded hydrogen atoms in one molecule,

   Mₐ₁M'ₐ₂D_{b1}D'_{b2}T_{c1}T'_{c2}Q_{d} (1)

   wherein
   M represents a siloxane unit represented by R¹₃SiO_{1/2},
   M' represents a siloxane unit represented by R¹₂HSiO_{1/2},
   D represents a siloxane unit represented by R²₂SiO_{2/2},
   D' represents a siloxane unit represented by R¹HSiO_{2/2},
   T represents a siloxane unit represented by R¹SiO_{3/2},
   T' represents a siloxane unit represented by HSiO_{3/2}, and
   Q represents a siloxane unit represented by SiO_{4/2},
   wherein R¹ independently represents a group selected from the group consisting of an alkyl group, aryl group, and aralkyl group having 1 to 30 carbon atoms; a1, a2, b1, b2, c1, c2, and d respectively represent integers of 0≤a1, 0≤a2, 0≤b1, 0≤b2, 0≤c1, 0≤c2, 0≤d, 2≤a2+b2+c2≤50, and 2≤a1+a2+b1+b2+c1+c2+d≤100,000, and
(A2) a linear organo(poly)siloxane represented by the following general formula (2) and having two or more alkenyl groups having 2 to 8 carbon atoms in one molecule,

   Mₑ₁M"ₑ₂D_{f1}D"_{f2} (2)

   wherein M and D are same as above,
   M" represents a siloxane unit represented by R¹₂R²SiO_{1/2}, and
   D" represents a siloxane unit represented by R¹R²SiO_{2/2},
   wherein R¹ is same as above; R² represents the alkenyl group having 2 to 8 carbon atoms; e1, e2, f1, and f2 respectively represent integers of 0≤e1≤2, 0≤e2≤2, 0≤f1, 0≤f2, e1+e2=2, 2≤e2+f2≤50, and 2≤e1+e2+f1+f2≤100,000.

Such a crosslinked organosiloxane of the component (A) has improved compatibility with an ultraviolet-ray absorbent and a highly polar oil, and thereby good feeling and makeup durability can be provided without impairing appearance when blended in a cosmetic.

In the general formula (2), it is preferable that f2=0.

Using such an alkenyl-group-containing organosiloxane of the component (A2) can smoothly carry out the polymerization reaction with the component (A1), which can yield a soft polymer (hydrosilylated reaction product) having a high thickening effect.

The component (A) is preferably a hydrosilylated reaction product of the following additional (A3),
(A3) a linear organohydrogen (poly)siloxane represented by the following general formula (3) and having one silicon-atom-bonded hydrogen atom in one molecule,

M_{g1}M'_{g2}Dₕ (3)

wherein M, M', and D are same as above; g1, g2, and h respectively represent integers of g1=g2=1 and 0≤h≤100.

The component (A) is preferably a hydrosilylated reaction product of the following additional (A4),
(A4) a linear organo(poly)siloxane represented by the following general formula (4) and having one alkenyl group having 2 to 8 carbon atoms in one molecule,

Mᵢ₁M"ᵢ₂Dⱼ (4)

wherein M, M", and D are same as above; i1, i2, and j respectively represent integers of i1=i2=1 and 0≤j≤100.

Such a configuration regulates a crosslinking density of the hydrosilylation reaction to provide appropriate properties of the component (A) according to a type of the cosmetic.

The inventive cosmetic preferably further comprises an oil agent (B) being liquid at 25°C in addition to the component (A).

The component (B) is preferably one or more selected from a modified silicone oil, a hydrocarbon oil, a fluorine-based oil, an ester oil, a natural animal or vegetable oil, and a semisynthetic oil.

Such a configuration yields good appearance and feel of use of the cosmetic, and easy handling.

In the inventive cosmetic, the component (A) and a part of the component (B) are preferably mixed in advance.

Since being resin-like or highly viscous gum-like product in many cases, the aromatic-group-modified crosslinked organosiloxane of the component (A) is preferably mixed with the liquid oil agent being the component (B) in advance to form a smooth paste, which facilitates easy blending in the cosmetic.

The inventive cosmetic preferably further comprises an ultraviolet-ray absorbent as a component (C).

Blending the ultraviolet-ray absorbent can yield a cosmetic having excellent ultraviolet-ray absorbing ability.

### ADVANTAGEOUS EFFECTS OF INVENTION

Since having improved compatibility with a highly polar oil, the aromatic-group-modified crosslinked organosiloxane (A) used in the inventive cosmetic can provide the cosmetic having an excellent thickening property and stability when blended in a cosmetic and having good feeling and coatability. In particular, the cosmetic having excellent stability can be provided even with blending with the ultraviolet-ray absorbent, which has known to have poor compatibility with a silicone.

### DESCRIPTION OF EMBODIMENTS

The present inventors have made earnest study to achieve the above object, and consequently found that the cosmetic having excellent compatibility with a highly polar oil can be obtained by using an aromatic-group-modified crosslinked organosiloxane having a specific structure. This finding has led to complete the present invention. This crosslinked organosiloxane can be synthesized without a special substituent, can easily improve the compatibility with a highly polar oil and an ultraviolet-ray absorbent, and eliminates necessity of a complex manufacturing step. Therefore, it is industrially useful means.

Specifically, the present invention is a cosmetic comprising the following component (A),
the component (A): a hydrosilylated reaction product of the following (A1) and (A2), the hydrosilylated reaction product being an aromatic-group-modified crosslinked organosiloxane, and a ratio between a total number of moles of aryl groups and aralkyl groups and a number of moles of silicon atoms is 0.34 or more,
(A1) an organohydrogen (poly)siloxane represented by the following general formula (1) and having two or more silicon-atom-bonded hydrogen atoms in one molecule,

   Mₐ₁M'ₐ₂D_{b1}D'_{b2}T_{c1}T'_{c2}Q_{d} (1)

   wherein
   M represents a siloxane unit represented by R¹₃SiO_{1/2},
   M' represents a siloxane unit represented by R¹₂HSiO_{1/2},
   D represents a siloxane unit represented by R¹₂SiO_{2/2},
   D' represents a siloxane unit represented by R¹HSiO_{2/2},
   T represents a siloxane unit represented by R¹SiO_{3/2},
   T' represents a siloxane unit represented by HSiO_{3/2}, and
   Q represents a siloxane unit represented by SiO_{4/2},
   wherein R¹ independently represents a group selected from the group consisting of an alkyl group, aryl group, and aralkyl group having 1 to 30 carbon atoms; a1, a2, b1, b2, c1, c2, and d respectively represent integers of 0≤a1, 0≤a2, 0≤b1, 0≤b2, 0≤c1, 0≤c2, 0≤d, 2≤a2+b2+c2≤50, and 2≤a1+a2+b1+b2+c1+c2+d≤100,000, and
(A2) a linear organo(poly)siloxane represented by the following general formula (2) and having two or more alkenyl groups having 2 to 8 carbon atoms in one molecule,

   Mₑ₁M"ₑ₂D_{f1}D"_{f2} (2)

   wherein M and D are same as above,
   M" represents a siloxane unit represented by R¹₂R²SiO_{1/2}, and
   D" represents a siloxane unit represented by R¹R²SiO_{2/2},
   wherein R¹ is same as above; R² represents the alkenyl group having 2 to 8 carbon atoms; e1, e2, f1, and f2 respectively represent integers of 0≤e1≤2, 0≤e2≤2, 0≤f1, 0≤f2, e1+e2=2, 2≤e2+f2≤50, and 2≤e1+e2+f1+f2≤100,000.

Hereinafter, embodiments of the present invention will be specifically described, but the present invention is not limited thereto.

### (A) Aromatic-Group-Modified Crosslinked Organosiloxane

The aromatic-group-modified crosslinked organosiloxane of the component (A), which is an essential component of the present invention, is a hydrosilylated reaction product of the following (A1) and (A2), the hydrosilylated reaction product being an aromatic-group-modified crosslinked organosiloxane, and a ratio between a total number of moles of aryl groups and aralkyl groups and a number of moles of silicon atoms is 0.34 or more,
(A1) an organohydrogen (poly)siloxane represented by the following general formula (1) and having two or more silicon-atom-bonded hydrogen atoms in one molecule,

   Mₐ₁M'ₐ₂D_{b1}D'_{b2}T_{c1}T'_{c2}Q_{d} (1)

   wherein
   M represents a siloxane unit represented by R¹₃SiO_{1/2},
   M' represents a siloxane unit represented by R¹₂HSiO_{1/2},
   D represents a siloxane unit represented by R²₂SiO_{2/2},
   D' represents a siloxane unit represented by R¹HSiO_{2/2},
   T represents a siloxane unit represented by R¹SiO_{3/2},
   T' represents a siloxane unit represented by HSiO_{3/2}, and
   Q represents a siloxane unit represented by SiO_{4/2},
   wherein R¹ independently represents a group selected from the group consisting of an alkyl group, aryl group, and aralkyl group having 1 to 30 carbon atoms; a1, a2, b1, b2, c1, c2, and d respectively represent integers of 0≤a1, 0≤a2, 0≤b1, 0≤b2, 0≤c1, 0≤c2, 0≤d, 2≤a2+b2+c2≤50, and 2≤a1+a2+b1+b2+c1+c2+d≤100,000, and
(A2) a linear organo(poly)siloxane represented by the following general formula (2) and having two or more alkenyl groups having 2 to 8 carbon atoms in one molecule,

   Mₑ₁M"ₑ₂D_{f1}D"_{f2} (2)

   wherein M and D are same as above,
   M" represents a siloxane unit represented by R¹₂R²SiO_{1/2}, and
   D" represents a siloxane unit represented by R¹R²SiO_{2/2},
   wherein R¹ is same as above; R² represents the alkenyl group having 2 to 8 carbon atoms; e1, e2, f1, and f2 respectively represent integers of 0≤e1≤2, 0≤e2≤2, 0≤f1, 0≤f2, e1+e2=2, 2≤e2+f2≤50, and 2≤e1+e2+f1+f2≤100,000.

Since having improved compatibility with an ultraviolet-ray absorbent and a highly polar oil, such a component (A) exhibits excellent compatibility, good feeling, and makeup durability when blended in a cosmetic.

In the general formula (1) of the component (A1), R¹ independently represents a group selected from an alkyl group, aryl group, and aralkyl group having 1 to 30 carbon atoms.

The alkyl group is preferably an alkyl group having 1 to 10, more preferably 1 to 6, carbon atoms. Examples of the alkyl group include: chain alkyl groups, such as a methyl group, an ethyl group, a propyl group, a butyl group, a lauryl group, a myristyl group, a palmityl group, and a stearyl group; and cycloalkyl groups, such as a cyclopentyl group and a cyclohexyl group. The alkyl group is preferably a methyl group or an ethyl group.

The aryl group is preferably an aryl group having 6 to 20, more preferably 6 to 10, carbon atoms. Examples of the aryl group include a phenyl group, a tolyl group, and a xylyl group. The aryl group is preferably a phenyl group.

The aralkyl group is preferably an aralkyl group having 7 to 20, more preferably 7 to 10, carbon atoms. Examples of the aralkyl group include a benzyl group and a phenethyl group. The aralkyl group is preferably a benzyl group.

In the general formula (1), a1 represents an integer of 0≤a1, preferably 0≤a1≤100, and more preferably 0≤a1≤50; a2 represents an integer of 0≤a2, preferably 0≤a2≤30, and more preferably 0≤a2≤10; b1 represents an integer of 0≤b1, more preferably 0≤b1≤5,000, and more preferably 0≤b1≤2,000; b2 represents an integer of 0≤b2, preferably 0≤b2≤50, and more preferably 0≤b2≤30; c1 represents an integer of 0≤c1, preferably 0≤c1≤100, and more preferably 0≤c1≤50; c2 represents an integer of 0≤c2, preferably 0≤c2≤30, and more preferably 0≤c2≤10; and d represents an integer of 0≤d, preferably 0≤d≤100, and more preferably 0≤d≤50.

Note that, 2≤a2+b2+c2≤50, preferably 2≤a2+b2+c2≤10, and more preferably 2≤a2+b2+c2≤5. If a2+b2+c2 is more than 50, a swelling property with a liquid oil agent and an ultraviolet-ray absorbent, and feeling as the cosmetic are deteriorated, which is unpreferable.

Furthermore, 2≤a1+a2+b1+b2+c1+c2+d≤100,000, preferably 2≤a1+a2+b1+b2+c1+c2+d≤10,000, and more preferably 2≤a1+a2+b1+b2+c1+c2+d≤2,000.

In the general formula (2) of the component (A2), R¹ independently represents a group selected from an alkyl group, aryl group, and aralkyl group having 1 to 30 carbon atoms. Specific examples and preferable aspects of each group can be selected from the same as those exemplified in the component (A1).

R² represents an alkenyl group having 2 to 8 carbon atoms, and for example, a monovalent hydrocarbon group having 2 to 8 carbon atoms and an unsaturated group at a terminal. Examples of R² include a vinyl group, an allyl group, a propenyl group, a butenyl group, a pentenyl group, and a hexenyl group. The alkenyl group is preferably a vinyl group in terms of easiness of the hydrosilylation reaction with the silicon-bonded hydrogen atom and good stability.

In the general formula (2), e1 represents an integer of 0≤e1≤2, e2 represents an integer of 0≤e2≤2, and e1+e2=2. In addition, f1 represents an integer of 0≤f1, preferably 0≤f1≤5,000, and more preferably 0≤f1≤1,000; and f2 represents an integer of 0≤f2, preferably 0≤f2≤50, and more preferably 0≤f2≤10. Note that, f2=0 is preferable because the component (A) becomes soft and has a high thickening effect.

Note that, 2≤e2+f2≤50, preferably 2≤e2+f2≤10, and more preferably 2≤e2+f2≤5. If e2+f2 is more than 50, a swelling property with a liquid oil agent and an ultraviolet-ray absorbent, and feeling as the cosmetic are deteriorated, which is unpreferable.

Furthermore, 2≤e1+e2+f1+f2≤100,000, more preferably 2≤e1+e2+f1+f2≤10,000, and more preferably 2≤e1+e2+f1+f2≤1,000.

In addition to the organohydrogen (poly)siloxane having two or more silicon-atom-bonded hydrogen atoms in one molecule represented by the general formula (1), a linear organohydrogen (poly)siloxane having one silicon-atom-bonded hydrogen atom in one molecule represented by the following general formula (3) may be added as a component (A3).
(A3) The following general formula (3)

M_{g1}M'_{g2}Dₕ (3)

In the formula, M, M', and D are same as described in the following formula (1). Each of g1, g2, and h represent integers of g1=g2=1 and 0≤h≤100, preferably 0≤h≤50.

A blending ratio between the component (A1) and the component (A3) (A1:A3) is not particularly decided, but preferably 0.1:99.9 to 99:1 at a mass ratio.

In addition to the organo(poly)siloxane having two or more alkenyl groups in one molecule represented by the general formula (2), a linear organo(poly)siloxane having one alkenyl group having 2 to 8 carbon atoms in one molecule represented by the following general formula (4) as a component (A4) may be added.
(A4) The following formula (4)

Mᵢ₁M"ᵢ₂Dⱼ (4)

In the formula, M, M", and D are same as described in the general formula (2). Each of i1, i2, and j represent i1=i2=1, and 0≤j≤100, preferably an integer of 0≤j≤50.

A blending ratio between the component (A2) and the component (A4) is not particularly decided, but preferably 0.1:99.9 to 99:1 at a mass ratio.

The inventive cosmetic has a feature of a ratio (X1/X2) between: a total number of moles (X1) of the aryl groups and the aralkyl groups in the crosslinked organosiloxane of the component (A); and a number of moles (X2) of the silicon atoms in the crosslinked organosiloxane of the component (A) being 0.34 or more. The mole ratio is more preferably within a range of 0.34 and 0.80. If the mole ratio is less than 0.34, the compatibility between the aromatic-group-modified crosslinked organosiloxane being the compound (A) and the highly polar liquid oil agent decreases, which fails to contain the oil agent at an amount equal to or higher than that of the organosiloxane, for example. Therefore, such a mole ratio is unpreferable because it deteriorates appearance or becomes a cause of stability deterioration when blended in the cosmetics.

The aromatic-group-modified crosslinked organosiloxane is modified with the aryl groups and the aralkyl groups therein at an amount so as to satisfy the above ratio as an entirety of the crosslinked product. In terms of efficient incorporation of the liquid oil agent and the ultraviolet-ray absorbent, the component (A2) and the component (A4) are preferably modified with the aryl groups and the aralkyl groups.

The ratio between: the total numbers of aromatic groups selected from the aryl groups and the aralkyl groups in the composition (A); and the number of the silicon atoms in the composition (A) is determined by the following calculation formula. For example, a case of a crosslinked product obtained by a hydrosilylation reaction between the following general formula (1-1) and the following general formula (2-1) will be described. 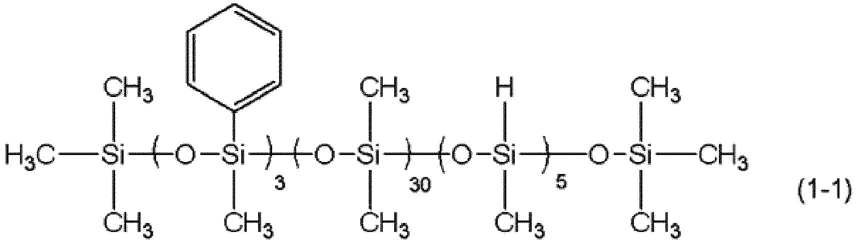
Phenyl groups: 3
Silicon atoms: 40
Reactive groups: 5

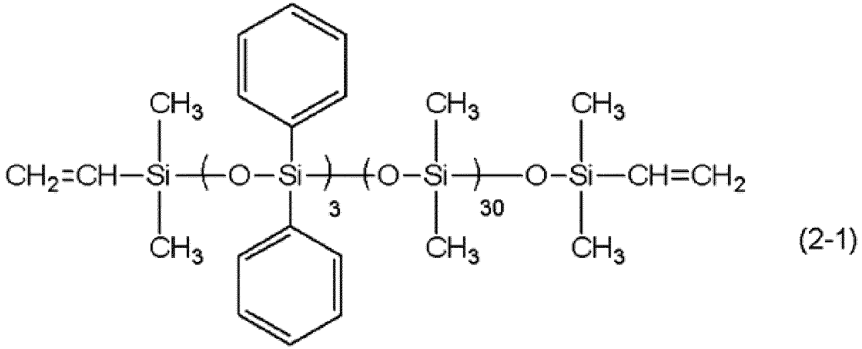

Phenyl groups: 6
Silicon atoms: 35
Reactive groups: 2
Calculation Formula: (3×2+6×5)/(40×2+35×5)=0.14

Here, a number of moles required for completing the hydrosilylation reaction is calculated as (1-1) : (2-1) = 2:5.

The aromatic-group-modified crosslinked organosiloxane (A) in the present invention is an aromatic-group-modified crosslinked organosiloxane obtained by reacting a silicone composition containing, for example, the component (A1) and the component (A2), and in some cases, the additional component (A3) and/or the component (A4) in the presence of a catalyst for a hydrosilylation reaction.

Here, the catalyst for a hydrosilylation reaction is not particularly limited, and examples thereof include: platinum compounds, such as chloroplatinic acid, an alcohol-modified chloroplatinic acid, and chloroplatinic acid-vinyl siloxane complex; or rhodium compounds. In the presence of the catalyst for a hydrosilylation reaction, the organohydrogensiloxanes of the component (A1) and the component (A3) and the alkenyl-group-containing organosiloxanes of the component (A2) and the component (A4) are reacted.

In this case, the reaction temperature is not particularly limited, but preferably 20 to 120°C. An amount of the added catalyst for a hydrosilylation reaction may be a known effective amount, and typically 0.1 to 500 ppm, preferably 0.5 to 200 ppm, and more preferably 1 to 100 ppm, in terms of platinum-group metal relative to a total amount of the organopolysiloxane mixture.

In this hydrosilylation reaction, a mole ratio of the wholly aliphatic unsaturated group / the hydrosilyl group is not particularly limited, but preferably 1/10 to 10/1, and more preferably 8/10 to 3/1.

The hydrosilylation reaction may be performed without a solvent, or an organic solvent or a volatile dimethylsilicone oil may be used as necessary.

Examples of such an organic solvent include: aliphatic alcohols, such as methanol, ethanol, 2-propanol, and butanol; aromatic hydrocarbons, such as benzene, toluene, and xylene; aliphatic or alicyclic hydrocarbons, such as n-pentane, n-hexane, and cyclohexane; halogenated hydrocarbons, such as dichloromethane, chloroform, and carbon tetrachloride; and ketone solvents, such as acetone and methyl ethyl ketone. From the viewpoint of use as cosmetics, solvent-free, ethanol, or 2-propanol is preferable.

Examples of the volatile dimethylsilicone oil include octamethylcyclotetrasiloxane, decamethylcyclopentasiloxane, dodecamethylcyclohexasiloxane, and dimethylpolysiloxane having a kinematic viscosity of 5 mm²/s or less.

Such an organic solvent or volatile dimethylsilicone oil can be removed by heating under a reduced pressure, etc. after the hydrosilylation reaction, as necessary. The hydrosilylation reaction may be performed in the presence of the component (B), and this method is most preferable in terms of usability and manufacturing.

The aromatic-group-modified crosslinked organopolysiloxane in the present invention is to improve the compatibility with the highly polar oil agent and the ultraviolet-ray absorbent, and its properties can be appropriately regulated according to the purpose of use. Specifically, there are spherical particle, formless particle, resin, non-flowable gum, flowable gum, and viscous liquid properties, and these can be observed visually, or with an optical microscope or an electron microscope. In terms of swelling property with the ultraviolet-ray absorbent or other oil agents, the structures other than a spherical particle shape are preferable.

About the feel of use and usability derived from a difference in the properties, blending a particle or resin aromatic-group-modified crosslinked organopolysiloxane in a cosmetic typically forms a cosmetic having an excellent soft-focusing property, moisty but not sticky, and silky and light feeling. The gum or liquid property is likely to yield a cosmetic having an excellent thickening effect, moisty feeling, and glossiness.

### (B) Oil Agent being Liquid at 25°C

An oil agent (B) being liquid at 25°C is preferably blended in the inventive cosmetic. In particular, the aromatic-group-modified crosslinked organopolysiloxane being the component (A) of the present invention is preferably mixed to be uniform with a part of the oil agent (B) being liquid at 25°C in advance. The aromatic-group modified crosslinked organopolysiloxane (A) used in the present invention is a highly viscous gum or a hard resin in many cases, and has a property of easy aggregation. Thus, mixing with the component (B) in advance facilitates direct blending in the cosmetic. In addition, uniformly dispersing the component (A) in the cosmetic is preferable in terms of good appearance and feel of use, and excellent stability.

The component (B) used in the present invention is preferably a liquid oil having a kinematic viscosity at 25°C of 0.65 to 10,000 mm²/s in terms of good operability, and preferably a liquid oil having good compatibility with an ultraviolet-ray absorbent (C), described later. In the present invention, the kinematic viscosity is measured by a method using a Cannon-Fenske Routine viscometer described in JIS Z 8803:2011. A type thereof is not particularly limited because of the purpose of facilitating the operability, and preferable example of the oil agent include the following modified silicone oils, hydrocarbon oils, fluorine-based oils, ester oils, natural animal or vegetable oils, and semisynthetic oils.

The modified silicone oil herein refers to a silicone oil other than Dimethicone (INCI), and specific examples thereof include: alkyl-modified silicones, such as Ethyl Methicone (INCI), Ethyltrisiloxane (INCI), and Hexyldimethicone (INCI); long-chain-alkyl-modified silicones, such as Caprylyl Methicone (INCI); linear or branched organopolysiloxanes, such as Phenyl Trimethicone (INCI), Diphenylsiloxy Phenyl Trimethicone (INCI), Diphenyl Dimethicone (INCI), and Tetraphenyldimethyldisiloxane (INCI); aromatic-group-modified silicones, such as cyclic organopolysiloxanes such as tetramethyltetraphenylcyclotetrasiloxane; aminomodified organopolysiloxanes, such as Amodimethicone (INCI) and Aminopropyl Dimethicone (INCI); pyrrolidone-modified orgagnopolysiloxanes, such as PCA Dimethicone (INCI); pyrrolidonecarboxylic-acid-modified organopolysiloxanes; amino-acid-modified silicones; and fluorine-modified silicones.

Examples of the hydrocarbon oil include a chain or cyclic hydrocarbon oil. Specific examples thereof include Olefin Oligomer (INCI), isoparaffins such as (C13,14) Isoparaffin (INCI); and alkanes such as Isododecane (INCI), Undecane (INCI), Dodecane (INCI), Isohexadecane (INCI), Hydrogenated Polyisobutene (INCI), Squalane (INCI), Mineral Oil (INCI), Coconut Alkane (INCI), and (C13-15) Alkane (INCI).

Examples of the fluorine-based oil include perfluoropolyether, perfluorodecaline, and perfluorooctane.

Examples of the ester oil include Diisobutyl Adipate (INCI), dihexyl decyl adipate (labeled name), Diheptylundecyl Adipate (INCI), n-alkyl glycol monoisostearates, such as Isostearyl Isostearate (INCI), Isocetyl Isostearate (INCI), Trimethylolpropane Triisostearate (INCI), Glycol Diethylhexanoate (INCI), Cetyl Ethylhexanoate (INCI), Trimethylolpropane Triethylhexanoate (INCI), Pentaerythrityl Tetraethylhexanoate (INCI), Cetyl Ethylhexanoate (INCI), octyldodecyl esters, such as Octyldodecyl Stearoyl Stearate (INCI), Oleyl Oleate (INCI), Octyldodecyl Oleate (INCI), Decyl Oleate (INCI), Neopentyl Glycol Diethylhexanoate (INCI), Neopentyl Glycol Dicaprate (INCI), Diisostearyl Malate (INCI), Triethyl Citrate (INCI), Diethylhexyl Succinate (INCI), Amyl Acetate (INCI), Ethyl Acetate (INCI), Butyl Acetate (INCI), Isocetyl Stearate (INCI), Butyl Stearate (INCI), Diisopropyl Sebacate (INCI), Diethylhexyl Sebacate (INCI), Cetyl Lactate (INCI), Myristyl Lactate (INCI), Isononyl Isononanoate (INCI), Isotridecyl Isononanoate (INCI), palmitate esters, such as Isopropyl Palmitate (INCI), Ethylhexyl Isopalmitate (INCI), Isocetyl Palmitate, and Hexyldecyl Palmitate (INCI), Cholesteryl Hydroxystearate (INCI), myristate esters, such as Isopropyl Myristate (INCI), Octyldodecyl Myristate (INCI), and Myristyl Myristate (INCI), Ethylhexyl Laurate (INCI), Hexyl Laurate (INCI), Dioctyldodecyl Lauroyl Glutamate (INCI), and Isopropyl Lauroyl Sarcosinate (INCI).

Among the ester oils, examples of glyceride oils include Triethyl Hexanoin (INCI), Caprylic/Capric Triglyceride (INCI), Coco-Glyceryl (INCI), Caprylic/Capric/Succinic Triglyceride (INCI), and (Caprylic/Capric) Glycerides (INCI).

Examples of the natural animal or vegetable oil agent and the semisynthetic oil agent include: natural vegetable oils, such as Persea Gratissima (Avocado) Oil (INCI), Linum Usitatissimum (Linseed) Seed Oil (INCI), Prunus Amygdalus Dulcis (Sweet Almond) Oil (INCI), perilla oil (labeled name), Olea Europaea (Olive) Fruit Oil (INCI), Torreya Californica (California Nutmeg) Oil (INCI), Cymbopogon Nardus (Citronella) Oil (INCI), Torreya Nucifera Seed Oil (INCI), Kyounin Yu (INCI), Triticum Vulgare (Wheat) Germ Oil (INCI), Sesamum Indicum (Sesame) Seed Oil (INCI), Triticum Vulgare (Wheat) Germ Oil (INCI), Oryza Sativa (Rice) Germ Oil (INCI), Oryza Sativa (Rice) Bran Oil (INCI), Camellia Kissi Seed Oil (INCI), Carthamus Tinctorius (Safflower) Seed Oil (INCI), Glycine Soja (Soybean) Oil (INCI), Camellia Sinensis Seed Oil (INCI), Camellia Japonica Seed Oil (INCI), Oenothera Biennis (Evening Primrose) Oil (INCI), RAPE SHUSHI YU (INCI), germ oils, such as Zea Mays (Corn) Germ Oil (INCI), and Triticum Vulgare (Wheat) Germ Oil (INCI), Persic Oil (labeled name), Elaeis Guineensis (Palm) Oil (INCI), Elaeis Guineensis (Palm) Kernel Oil (INCI), Ricinus Communis (Castor) Seed Oil (INCI), Helianthus Annuus (Sunflower) Seed Oil (INCI), Vitis Vinifera (Grape) Seed Oil (INCI), Simmondsia Chinensis (Jojoba) Seed Oil (INCI), Macadamia Ternifolia Seed Oil (INCI), Limnanthes Alba (Meadowfoam) Seed Oil (INCI), Gossypium Herbaceum (Cotton) Seed Oil (INCI), Cocos Nucifera (Coconut) Oil (INCI), and peanut oil; natural animal oils, such as Shark Liver Oil (INCI), Cod Liver Oil (INCI), Fish Liver Oil (INCI), Turtle Oil (INCI), Mink Oil (INCI), and Egg Oil (INCI); and semisynthetic oils and fats, such as Hydrogenated Coconut Oil (INCI) and Liquid Lanolin (INCI).

Among the above components (B), the modified silicone oil and the ester oil are preferable, and particularly preferable modified silicone oils are linear or branched organopolysiloxanes, such as Phenyl Trimethicone (INCI), which is a silicone oil having a phenyl group, Diphenylsiloxy Phenyl Trimethicone (INCI), Diphenyl Dimethicone (INCI), and a dimethylsiloxanemethylphenylsiloxane copolymer. Examples of commercial products thereof include diphenyl dimethicone (KP-54, KP-54HV, etc., manufactured by Shin-Etsu Chemical Co., Ltd.) and diphenylsiloxy phenyl trimethicone (KP-56A, manufactured by Shin-Etsu Chemical Co., Ltd.).

Particularly preferable ester oils are Triethyl Hexanone (INCI), Isononyl Isononanoate (INCI), Isotridecyl Isononanoate (INCI). Cetyl Ethylhexanoate (INCI), Caprylic/Capric Triglyceride (INCI), and Ethylhexyl Isopalmitate (INCI).

A mixing ratio between the component (A) and the component (B) (the component (A) / the component (B)) is not particularly limited, but preferably 1/30 to 30/1 (mass ratio), and more preferably 1/20 to 10/1. The mixing method is not particularly limited, and known methods such as a triple-roller mill, a double-roller mill, a kneader, a mass colloider, a sand grinder, a colloid mill, a Gaulin homogenizer, a Disper, and a high-shear mixer can be used. To form a paste composition with smooth appearance, a method with a triple-roll miller or a Disper is preferable.

### Ultraviolet-Ray Absorbent (C)

An ultraviolet-ray absorbent (C) is preferably blended in the inventive cosmetic using the aromatic-group-modified crosslinked organosiloxane. Since the aromatic-group-modified crosslinked organosiloxane of the component (A) exhibits high compatibility with the ultraviolet-ray absorbent, a cosmetic having excellent stability can be prepared.

Examples of the ultraviolet-ray absorbent of the component (C) include Homosalate, Octocrylene (INCI), Butyl Methoxydibenzoylmethane (INCI), Ethylhexyl Salicylate (INCI), Diethylamino Hydroxybenzoyl Hexyl Benzoate (INCI), Benzophenone-6 (INCI), Benzophenone-9 (INCI), Benzophenone-1 (INCI), Polysilicone-15 (INCI), Ethylhexyl Dimethoxybenzylidene Dioxoimidazolidine Propionate (INCI), Benzophenone-2 (INCI), Terephthalylidene Dicamphor Sulfonic Acid (INCI), Ethylhexyl Triazone (INCI), Isopentyl Trimethoxycinnamate Trisiloxane (INCI), Drometrizole Trisiloxane (INCI), Ethylhexyl Dimethyl PABA (INCI), Isopropyl Methoxycinnamate (INCI), Ethylhexyl Methoxycinnamate (INCI), Bis-Ethylhexyloxyphenol Methoxyphenyl Triazine (INCI), Benzophenone-3 (INCI), Benzophenone-4 (INCI), Benzophenone-5 (INCI), Phenylbenzimidazole Sulfonic Acid (INCI), Methylene Bis-Benzotriazolyl Tetramethylbutylphenol (INCI), Glyceryl Ethylhexanoate Dimethoxycinnamate (INCI), Glyceryl PABA (INCI), Diisopropyl Methyl Cinnamate (INCI), Cinoxate (INCI), and Ethylhexyl Dimethoxybenzylidene Dioxoimidazolidine Propionate (INCI).

A UVA absorbent (such as, for example, Diethylamino Hydroxybenzoyl Hexyl Benzoate (INCI)) and a UVB absorbent (such as, for example, Ethylhexyl Methoxycinnamate (INCI)) can be used in combination, or each of them can be freely combined.

Among these, one or two or more organic ultraviolet-ray absorbents selected from ethylhexyl methoxycinnamate, hexyl diethylaminohydroxybenzoylbenzoate, ethylhexyl salicylate, polysilicone-15, t-butylmethoxydibenzoylmethane, oxybenzone, methylenebisbenzotriazolyltetramethylbutylphenol, bis-ethylhexyloxyphenol methoxyphenyl triazine, homomenthyl salicylate, and octocrylene are particularly preferable from the viewpoint of compatibility with the oil agent.

The aromatic-group-modified crosslinked organosiloxane of the component (A) has high compatibility with the component (C) and has a high thickening property, and is excellence in stabilizing the cosmetic containing the component (C) and improving the feel of use. A blending amount of the component (C) is not particularly limited, but preferably 0.1 mass% to 35 mass%, more preferably 5 mass% to 27 mass%, further preferably 7 mass% to 20 mass%, and most preferably 10 mass% to 20 mass%, in an entirety of the cosmetic. The present invention is particularly preferable for stabilizing a system in which the component (C) is blended at a large amount, which destabilizes the cosmetic.

### <Other Components>

In the inventive cosmetic, the following components that can be blended in a cosmetic can be blended as other components. These can be used singly, or two or more thereof can be appropriately combined to use. These components are appropriately selected and used according to a type of the cosmetic, etc., and a blending amount thereof can also be a known blending amount according to the type of the cosmetic, etc.

### -Aqueous Component

An aqueous component is not particularly limited as long as it is an aqueous component that can be commonly blended in a cosmetic. Specific examples thereof include a moisturizer such as: water; lower alcohols, such as ethanol; sugar alcohols, such as erythritol, maltitol, xylitol, and sorbitol; and polyhydric alcohols, such as 1,3-BG, glycerin, PG, DPG, and pentylene glycol. These can be used singly, or two or more can be appropriately combined to use. When this component is blended, the blending amount is preferably 0.1 to 90 mass% in the cosmetic.

### -Oily Component Other Than Component (B)

An oily component other than the component (B) is not particularly limited as long as it is used for a common cosmetic. Examples thereof include: esters being a paste at 25°C, Vaseline, and lanolin; solid waxes; and silicone waxes.

### -Powder

A powder is not particularly limited as long as it is a raw material that can be commonly blended in a cosmetic. Examples thereof include coloring pigments, inorganic powders, organic powders, inorganic-organic composite powders, and metal powders. When the powder is blended, a blending mount of the powder is not particularly limited, but blended at preferably 0.1 to 90 mass%, more preferably at 1 to 35 mass%, further preferably at 1 to 18 mass%, in the entirety of the cosmetic.

Usable as the coloring pigment is any of red iron oxide, yellow iron oxide, white titanium oxide, black iron oxide, colcothar, ultramarine, iron blue, manganese violet, cobalt violet, chromium hydroxide, chromium oxide, cobalt oxide, cobalt titanate, iron-oxide-doped titanium oxide, iron titanate, a (titanium / titanium oxide) sintered product, (Li/cobalt) titanate, cobalt titanate, titanium nitride, iron hydroxide, inorganic brown pigments such as γ-iron oxide, inorganic yellow pigments such as ocher, and colored pigments such as lake of tar pigments and lake of natural pigments.

Examples of the inorganic powder include fine particles composed of zirconium oxide, zinc oxide, cerium oxide, magnesium oxide, barium sulfate, calcium sulfate, magnesium sulfate, calcium carbonate, magnesium carbonate, talc, cleaving talc, mica, kaolin, sericite, muscovite, synthetic mica, phlogopite, lepidolite, biotite, lithia mica, silicic acid, silicon dioxide, fumed silica, hydrous silicon dioxide, aluminum silicate, magnesium silicate, aluminum magnesium silicate, calcium silicate, barium silicate, strontium silicate, tungstate metal salts, hydroxyapatite, vermiculite, heidilite, bentonite, montmorillonite, hectorite, zeolite, ceramics, dibasic calcium phosphate, alumina, aluminum hydroxide, boron nitride, glass, etc. Examples of inorganic colored pearl pigments include pearl pigments such as titanium-oxide-coated mica, bismuth oxychloride, titanium-oxide-coated bismuth oxychloride, titanium-oxide-coated talc, fish scales, and titanium-oxide-coated colored mica. Examples thereof for use of ultraviolet-ray shielding include titanium oxide fine particles, zinc oxide fine particles, and cerium oxide fine particles. Titanium oxide fine particles and zinc oxide fine particles are preferable in terms of ultraviolet-ray shielding and usability. These inorganic powders for shielding ultraviolet ray can be used alone or in combination, and appropriately selected according to the absorbing wavelength, dispersibility, etc.

A blending amount of the inorganic powder for use of ultraviolet-ray shielding is not particularly limited, but blended preferably at 0.1 to 35 mass%, more preferably at 1 to 18 mass%, further preferably at 1 to 10 mass%, and most preferably at 1 to 5 mass%, in the entirety of the cosmetic. Since the component (A) in the present invention has high compatibility with the component (C), the component (C) can be blended at a large amount in the cosmetic. Thus, the component (C) can be blended at a large amount in order to obtain a desired ultraviolet-ray shielding effect, and meanwhile the blending amount of the inorganic powder for use of ultraviolet-ray shielding can be reduced, which enables to inhibit creaking feel and whiteness that are characteristics of the inorganic powder to achieve preparation of a sunscreen cosmetic with high clearness. Alternatively, according to a desired ultraviolet-ray shielding effect, the component (C) can be blended at a large amount and the inorganic powder for use of ultraviolet-ray shielding can be additionally blended, which can achieve preparation of a stable cosmetic having a higher ultraviolet-ray shielding effect.

Examples of the organic powder include powders composed of a silicone, a polyamide, a polyacrylic acidacrylate ester, a polyester, polyethylene, polypropylene, polystyrene, a styrene-acrylic acid copolymer, a divinylbenzene-styrene copolymer, a polyurethane, a vinyl resin, an urea resin, a melamine resin, benzoguanamine, a polymethylbenzoguanamine, tetrafluoroethylene, polymethyl methacrylate (for example, methyl polymethacrylate), cellulose, silk, nylon, a phenol resin, an epoxy resin, a polycarbonate, etc. In particular, examples of the silicone include silicone resin particles (specific examples thereof include KMP-590, KMP-591, and KMP-592, manufactured by Shin-Etsu Chemical Co., Ltd.), silicone rubber powders (specific examples thereof include KMP-597 and KMP-598, manufactured by Shin-Etsu Chemical Co., Ltd.), and silicone-resin-coated silicone rubber powders (specific examples thereof include KSP-100, KSP-101, KSP-102, KSP-105, KSP-300, KSP-411, and KSP-441, manufactured by Shin-Etsu Chemical Co., Ltd.), and these may be dispersed in water or oil in advance (specific examples thereof include KM-9729 and KSG-016F, manufactured by Shin-Etsu Chemical Co., Ltd.). Examples thereof also include metal soaps, and specific examples thereof include powders composed of zinc stearate, aluminum stearate, calcium stearate, magnesium stearate, zinc myristate, magnesium myristate, zinc cetyl phosphate, calcium cetyl phosphate, zinc sodium cetyl phosphate, etc. Examples thereof further include organic pigments, and specific examples thereof include: tar pigments, such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural pigments, such as carminic acid, laccaic acid, carthamin, brazilin, and crocin.

Examples of the inorganic-organic composite powder include a composite powder in which a surface of an inorganic powder is coated with an organic powder by a known used method.

Examples of the metal powder include metal fine particles composed of aluminum, copper, stainless steel, silver, etc.

The above powders can be subjected to a treatment of the particle surface to use. Preferable surfacetreating agent therefor is that can impart hydrophobicity from the viewpoint of water resistance of the cosmetic. This treating agent for imparting hydrophobicity is not particularly limited, and examples thereof include silicone treating agents, waxes, paraffins, organic fluorine compounds such as a perfluoroalkyl phosphate salt, surfactants, amino acids such as N-acylglutamic acid, and metal soaps such as aluminum stearate and magnesium myristate.

The treating agent is more preferably the silicone treating agent. Examples thereof include: silanes or silylating agents such as caprylsilane (AES-3083, manufactured by Shin-Etsu Chemical Co., Ltd.) or trimethoxysilyl dimethicone; silicone oils, such as dimethylsilicone (KF-96A series, manufactured by Shin-Etsu Chemical Co., Ltd.), methylhydrogen-type polysiloxanes (such as KF-99P and KF-9901, manufactured by Shin-Etsu Chemical Co., Ltd.), and silicone-branched silicone treating agents (such as KF-9908 and KF-9909, manufactured by Shin-Etsu Chemical Co., Ltd.); and acrylsilicones (KP-574 and KP-541, manufactured by Shin-Etsu Chemical Co., Ltd.). Furthermore, the above surface-hydrophobizing treating agent can be used singly, or used in combination of two or more thereof.

Among the silicone treating agents, triethoxysilyl ethylpolydimethylsiloxy ethylhexyl dimethicone, which is a dimethylpolysiloxane having a triethoxysilyl group, a polydimethylsiloxyethyl group, and a hexyl group on the side chain, (KF-9909, manufactured by Shin-Etsu Chemical Co., Ltd.), etc. can be effectively used in terms of exhibiting high compatibility even when a dispersion medium to disperse the highly surface-hydrophobized colored pigment according to the present invention is a mixed composition of the silicones and a hydrocarbon, etc. Furthermore, the above surface-hydrophobizing treating agent can be used singly, or used in combination of two or more thereof. Specific examples of the colored pigment subjected to the surface-hydrophobizing treatment include KTP-09 series, specifically, KTP-09W, KTP-09R, KTP-09Y, and KTP-09B (manufactured by Shin-Etsu Chemical Co., Ltd.).

### -Surfactant

Surfactants include nonionic, anionic, cationic, and amphoteric surfactants. The surfactant is not particularly limited, and any one can be used as long as it is used for a common cosmetic. Among these surfactants, preferable surfactants are a partially crosslinked polyether-modified silicone, a partially crosslinked polyglycerin-modified silicone, a linear or branched polyoxyethylene-modified organopolysiloxane, a linear or branched polyoxyethylene-polyoxypropylenemodified organopolysiloxane, a linear or branched polyoxyethylene-alkyl-co-modified organopolysiloxane, a linear or branched polyoxyethylene-polyoxypropylenealkyl-co-modified organopolysiloxane, a linear or branched polyglycerin-modified organopolysiloxane, a linear or branched polyglycerin-alkyl-co-modified organopolysiloxane, and a linear or branched pyrrolidone-modified organopolysiloxane.

In these surfactants, a content of a hydrophilic polyoxyethylene group, polyoxyethylene-polyoxypropylene group, or a polyglycerin residual group is preferably 10 to 70 mass% in the molecule.

When the crosslinked organopolysiloxane such as the partially crosslinked polyether-modified silicone and the partially crosslinked polyglycerin-modified silicone is used, the crosslinked organopolysiloxane is preferably swelled, in a composition composed of the crosslinked organopolysiloxane and an oil agent being liquid at a room temperature, by containing the liquid oil agent having a weight equal to or larger than that of the crosslinked organopolysiloxane. For the liquid oil agent, the liquid silicone, the hydrocarbon oil, the ester oil, the natural animal or vegetable oil, the semisynthetic oil, and the fluorine-based oil, which are in the oil agent of the component (B), can be used. Examples thereof include: a low-viscous silicone with 0.65 to 100 mm²/s (25°C); hydrocarbon oils, such as a liquid paraffin, squalane, isododecane, and isohexadecane; glyceride oils, such as trioctanoin; ester oils, such as isotridecyl isononanoate, an N-acylglutamate ester, and a lauroylsarcosinate ester; and a natural animal or vegetable oil such as macadamia nut oil.

Specific examples of the swelled crosslinked organopolysiloxane containing the liquid oil agent include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, and KSG-850Z, manufactured by Shin-Etsu Chemical Co., Ltd.

Specific examples of surfactants not being the crosslinked organopolysiloxane include KF-6011, KF-6013, KF-6043, KF-6017, KF-6028, KF-6038, KF-6048, KF-6100, KF-6104, KF-6105, and KF-6106, manufactured by Shin-Etsu Chemical Co., Ltd.

In any cases, a blending amount of the surfactant is preferably 0.1 to 20 mass% in the entirety of the cosmetic. The blending amount of 0.1 mass% or more can sufficiently achieve functions of dispersion and emulsification, and the blending amount of 20 mass% or less has no risk of sticky feel of use of the cosmetic, which are preferable. HLB of the surfactant is not limited but preferably 2 to 14.5 for a purpose of keeping the water resistance of the cosmetic.

### -Crosslinked Organopolysiloxane Other Than Component (A)

In the present invention, a crosslinked organopolysiloxane other than the component (A) can be blended. Such a crosslinked organopolysiloxane is not particularly limited as long as it is commonly used for cosmetics, and can be used singly, or two or more thereof can be appropriately combined to use. This crosslinked organopolysiloxane does not have a spherical shape, which differs from the above powder. This crosslinked organopolysiloxane is a compound having no polyether nor polyglycerin structure in the molecular structure, which differs from the above surfactant. This crosslinked organopolysiloxane is an elastomer to have a structural viscosity by swelling with the component (B).

Specific examples thereof include (Dimethicone / Vinyl Dimethicone) Crosspolymer, (Dimethicone / Phenyl Vinyl Dimethicone) Crosspolymer, (Vinyl Dimethicone / Lauryl Dimethicone) Crosspolymer, and (Lauryl Polydimethylsiloxy Ethyl Dimethicone / Bisvinyl Dimethicone) Crosspolymer, defined as a cosmetic labeled name. These are commercially available as a swelled product containing oil being liquid at a room temperature, and specific examples thereof include KSG-15, KSG-1510, KSG-16, KSG-1610, KSG-18A, KSG-19, KSG-41A, KSG-42A, KSG-43, KSG-44, KSG-042Z, KSG-045Z, and KSG-048Z, manufactured by Shin-Etsu Chemical Co., Ltd. When this component is blended, the blending amount is preferably 0.01 to 30 mass% as a solid content in the cosmetic.

### -Coating Agent

A coating agent is not particularly limited as long as it is a raw material that can be commonly blended in a cosmetic. Specific usable coating agents are polyvinyl alcohol, polyvinylpyrrolidone, polyvinyl acetate, latexes of a polyalkyl acrylate, etc., dextrin, cellulose derivatives such as an alkylcellulose and nitrocellulose, silicone-polysaccharide compounds such as tri(trimethylsiloxy)silylpropylcarbamate pullulan, acryl-silicone-based grafted copolymers such as an (alkyl acrylate / dimethicone) copolymer, silicone resins such as trimethylsiloxylsilicic acid, silicone-modified polynorbornene, silicone-based resins such as a fluorine-modified silicone resin, fluororesins, aromatic hydrocarbon resins, polymer emulsion resins, terpenebased resins, polybutene, polyisoprene, alkyd resins, polyvinylpyrrolidone-modified polymers, rosin-modified resins, and polyurethanes.

Among these, the silicone coating agent is particularly preferable, and usable are, not limited to, tri(trimethylsiloxy)silylpropylcarbamate pullulan (the commercial product dissolved in a solvent are TSPL-30-D5 and ID, manufactured by Shin-Etsu Chemical Co., Ltd.), an (alkyl acrylate / dimethicone) copolymer (the commercial product dissolved in a solvent are KP-543, KP-545, KP-549, KP-550, KP-545L, etc., manufactured by Shin-Etsu Chemical Co., Ltd.), trimethylsiloxysilicic acid (the commercial product dissolved in a solvent are KF-7312J, X-21-5250, etc., manufactured by Shin-Etsu Chemical Co., Ltd.), a silicone-modified polynorbornene (the commercial product dissolved in a solvent are NBN-30-ID, etc., manufactured by Shin-Etsu Chemical Co., Ltd.), and an organosiloxane-grafted polyvinyl alcohol polymer. The coating agent may be singly, or two or more thereof may be used. When this component is blended, the blending amount is preferably 0.1 to 20 mass% in the cosmetic.

### -Other Additives

Examples of other additives include an oil-soluble gelling agent, a water-soluble thickener, an antiperspirant, an antiseptic and antibacterial agent, a perfume, salts, an oxidation inhibitor, a pH regulator, a chelating agent, a refreshing agent, an anti-inflammatory agent, skin-beautifying components (such as a whitening agent, a cell activator, a rough skin improver, a blood circulation promoter, a skin astringent, and an anti-seborrhoea agent), vitamins, amino acids, a nucleic acid, a hormone, and a clathrate compound.

### -Oil-Soluble Gelation Agent

Examples of the oil-soluble gelation agent include: metal soaps, such as aluminum stearate, magnesium stearate, and zinc myristate; amino acid derivatives, such as N-lauroyl-L-glutamic acid and α,γ-di-n-butylamine; dextrin fatty acid esters, such as dextrin palmitate ester, dextrin stearate ester, and dextrin 2-ethylhexanoate palmitate ester; sucrose fatty acid esters, such as sucrose palmitate ester and sucrose stearate ester; fructo-oligosaccharide fatty acid esters, such as fructo-oligosaccharide stearate ester and fructo-oligosaccharide 2-ethylhexanoate ester; sorbitol benzylidene derivatives, such as monobenzylidene sorbitol and dibenzylidene sorbitol; and organicmodified clay minerals, such as disteardimonium hectorite, stearalkonium hectorite, and hectorite.

### -Water-Soluble Thickener

Examples of the water-soluble thickener include: vegetable polymers, such as gum Arabic, tragacanth, galactan, carob gum, guar gum, karaya gum, carrageenan, pectin, agar, quince seed (marmelo), starch (rice, corn, potato, wheat, etc.), algae colloid, trant gum, and Locust bean gum; microorganism polymers, such as xanthan gum, dextran, succinoglucan, and pullulan; animal polymers, such as collagen, casein, albumin, and gelatin; starch polymers, such as carboxymethylstarch and methylhydroxypropylstarch; cellulose polymers, such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose, a cationized cellulose, and a cellulose powder; alginate polymers, such as sodium alginate and propylene glycol alginate ester; vinyl polymers, such as polyvinyl methyl ether and a carboxyvinyl polymer; polyoxyethylene polymers; polyoxyethylene-polyoxypropylene copolymers; acrylic polymers, such as sodium polyacrylate, polyethyl acrylate, polyacrylamide, and an acryloyl dimethyl taurine salt copolymer; other synthetic water-soluble polymers, such as a polyethyleneimine and a cation polymer; and inorganic water-soluble polymers, such as bentonite, aluminum magnesium silicate, montmorillonite, beidellite, nontronite, saponite, hectorite, and silicic anhydride.

Among these, preferably usable are one or more water-soluble thickeners selected from vegetable polymers, microorganism polymers, animal polymers, starch polymers, cellulose polymers, alginate polymers, a polyoxyethylene-polyoxypropylene copolymer, acrylic polymers, and inorganic water-soluble polymers.

### -Antiperspirant

Examples of the antiperspirant include: aluminum hydroxyhalides, such as chlorohydroxyaluminum and allantoin chlorohydroxyaluminum; aluminum halides, such as aluminum chloride; allantoin aluminum salts, tannic acid, persimmon tannin, aluminum potassium sulfate, zinc oxide, zinc paraphenolsulfonate, burnt alum, tetrachloro (Al/zirconium) hydride, and trichlorohydrex glycine (Al/zirconium). In particular, preferable for yielding a high effect are aluminum hydroxyhalides, aluminum halides, and a complex or mixture thereof with zirconyl oxyhalide and zirconyl hydroxyhalide (for example, tetrachloro (Al/zirconium) hydride and trichlorohydrex glycine (Al/zirconium)).

### -Antiseptic and Antibacterial Agent

Examples of the antiseptic and antibacterial agent include a paraoxybenzoic acid alkyl ester, benzoic acid, sodium benzoate, sorbic acid, potassium sorbate, phenoxyethanol, imidazolidinylurea, salicylic acid, isopropylmethylphenol, carbolic acid, parachlorometacresol, hexachlorophene, benzalkonium chloride, chlorhexidine chloride, trichlorocarbanilide, iodopropynyl butylcarbamate, polylysine, a photosensitizer, silver, and a vegetable extract.

### -Perfume

The perfume includes natural perfumes and synthetic perfumes. Examples of the natural perfumes include: vegetable perfumes isolated from flowers, leaves, wood, pericarps, etc.; and animal perfumes, such as musk and civet. Examples of the synthetic perfumes include: hydrocarbons, such as a monoterpene; alcohols, such as an aliphatic alcohol and an aromatic alcohol; aldehydes, such as a terpene aldehyde and an aromatic aldehyde; ketones, such as an alicyclic ketone; esters, such as a terpene ester; lactones, phenols, oxides, nitrogencontaining compounds, and acetals.

### -Salts

Examples of the salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and amino acid salts include: salts of amines, such as triethanolamine; and salts of amino acids, such as glutamic acid. In addition, salts of hyaluronic acid, chondroitin sulfuric acid, and neutralized salts of an acid and a base used in pharmaceutical formulation can also be used.

### -Oxidation Inhibitor

The oxidation inhibitor is not particularly limited, and examples thereof include a carotinoide, ascorbic acid and a salt thereof, ascorbyl stearate, tocophenol, tocophenyl acetate, tocopherol, p-t-butylphenol, butylhydroxyanisole, dibutylhydroxytoluene, phytic acid, ferulic acid, thiotaurine, hypotaurine, a sulfite salt, erythorbic acid and a salt thereof, chlorogenic acid, epicatechin, epigallocatechin, epigallocatechin gallate, apigenin, kaempferol, myricetin, and quercetin.

### -pH Regulator

Examples of the pH regulator include lactic acid, citric acid, glycolic acid, succinic acid, tartaric acid, dl-malic acid, potassium carbonate, sodium hydrogen carbonate, and ammonium hydrogen carbonate.

### -Chelating Agent

Examples of the chelating agent include alanine, sodium edetate salt, sodium polyphosphate, sodium metaphosphate, and phosphoric acid.

### -Refreshing Agent

Examples of the refreshing agent include L-menthol, camphor, and menthyl lactate.

### -Anti-Inflammatory Agent

Examples of the anti-inflammatory agent include allantoin, glycyrrhizic acid and a salt thereof, glycyrrhetinic acid and stearyl glycyrrhetinate, tranexamic acid, and azulene.

### -Skin-Beautifying Component

Examples of the skin-beautifying component include: whitening agents, such as a placenta extract, arbutin, glutathione, tranexamic acid, an ascorbic acid derivative, and a Saxifraga stolonifera extract; cell activators, such as royal jelly, a photosensitizer, a cholesterol derivative, and a calf blood extract; rough skin improvers; blood circulation promoters, such as nonylwallenylamide, nicotinic acid benzyl ester, nicotinic acid β-butoxyethyl ester, capsaicin, zingerone, cantharides tincture, ichthammol, caffeine, tannic acid, α-borneol, tocopheryl nicotinate, inositol hexanicotinate, cyclandelate, cinnarizine, tolazoline, acetylcholine, verapamil, cepharanthine, and γ-oryzanol; skin astringents; and anti-seborrhoea agents, such as sulfur and thiantrol.

### -Vitamins

Examples of the vitamins include: vitamin A, such as vitamin A oil, retinol, retinyl acetate, and retinyl palmitate; vitamin B, such as vitamin B2, such as riboflavin, riboflavin butyrate, and flavin adenine nucleotide, vitamin B6, such as pyridoxine hydrochloride salt, pyridoxine dioctanoate, and pyridoxine tripalmitate, vitamin B12 and a derivative thereof, and vitamin B15 and a derivative thereof; vitamin C, such as L-ascorbic acid, L-ascorbic acid dipalmitate ester, L-ascorbic acid-2-sodium sulfate, and L-ascorbic acid dipotassium phosphate diester; vitamin D, such as ergocalciferol and cholecalciferol; vitamin E, such as α-tocopherol, β-tocopherol, γ-tocopherol, dl-α-tocopheryl acetate, dl-α-tocopheryl nicotinate, and dl-α-tocopheryl succinate; nicotinic acids, such as nicotinic acid, benzyl nicotinate, and nicotinamide; vitamin H; vitamin P; pantothenic acids, such as calcium pantothenate, D-pantothenyl alcohol, pantothenyl ethyl ether, and acetylpantothenyl ethyl ether; and biotin.

### -Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

### -Nucleic acid

Examples of the nucleic acid include a deoxyribonucleic acid.

### -Hormone

Examples of the hormone include estradiol and ethenylestradiol.

### -Chelate Compound

Examples of the chelate compound include cyclodextrin.

### <Cosmetic>

The aromatic-group-modified crosslinked organosiloxane (A) in the present invention can be used for cosmetics in various use, and particularly applicable as a raw material of all cosmetics for external use for skin and hair. In this case, a blending amount of the component (A) is preferably within a range of 0.01 to 10 mass%, and further preferably within a range of 0.05 to 2 mass% in the entirety of the cosmetic. The amount of 0.01 mass% or more can yield a sufficient thickening property, and the amount of 10 mass% or less yields good feel of use.

A form of the aforementioned cosmetic may be any of emulsion, non-aqueous, and aqueous systems. For imparting fresh feel of use, an emulsion form is selected, and the emulsion form may be any of O/W emulsion, W/O emulsion, O/W/O emulsion, and W/O/W emulsion. For obtaining oily feel or water resistance, a non-aqueous composition or a powder composition can be selected. For obtaining plain feel of use, an aqueous composition can be selected. A good cosmetic can be obtained in any cases. In the present invention, "non-aqueous composition" refers to a composition in which water is intentionally not blended, and "aqueous composition" refers to a composition in which an oily component is intentionally not blended. Among these, the W/O emulsion and the non-aqueous composition with which a particularly high thickening property is expected are preferable. Examples of the W/O emulsion include a bilayer separable composition, which is shaken well before use, and an inseparable composition, which can be used as it is without shaking. The bilayer separable composition include several types, but refers to, in a broad sense, those in which an emulsion composition or a dispersion separates into a plurality of phases with leaving to stand. For example, the bilayer separable composition include, with leaving to stand: those separate into two layers of a powder-containing layer and an emulsion layer; those separate into two layers of a powder-containing layer and an oil layer; those in which an aqueous layer and an oil layer are separated; those in which an emulsion and a part of an aqueous layer are separated; those in which an emulsion containing a powder and a part of an aqueous layer are separated; and those in which a layer composed of a powder and an oil layer component, and an aqueous layer are separated. All of these compositions have a feature of forming emulsified and dispersed, or dispersed one layer immediately after shaking. To easily mix these, some formulations contain stainless balls in the container. These bilayer separable types are also called as "bilayer type", "shaking type", "rustle-rustle type (which may be used in spoken language)", etc. The W/O emulsion according to the present invention exhibits the effect of the present invention as the bilayer separable type, which is well shaken before use, or as the inseparable type, which can be used as it is without shaking, but is preferably the inseparable type, which needs no shaking, in terms of usability.

The inventive cosmetic using the aromatic-group-modified crosslinked organosiloxane can be formulated by various methods. For example, the cosmetic may be mixed with an oil agent, an ultraviolet-ray absorbent, or a powder. In a case of an emulsified composition, the cosmetic may be mixed with an oil phase before emulsifying, an emulsion after emulsifying, or both thereof. The mixing may be performed in a step at a room temperature or in a heating step. The mixing method is not particularly limited, and a pulsator, a propeller mixer, a puddle mixer, a Disper, a homogenizing mixer, a roller, a Henschel mixer, an atomizer, a pin mill, a rotation/revolution mixer, etc. may be used.

The cosmetic in the present invention is not particularly limited, and can be applied for various products such as, for example, cosmetic essence, milky lotion, cream, hair care, foundation, cosmetic base, sunscreen, concealer, cheek color, lipstick, gloss, balm, mascara, eye shadow, eye liner, body make-up, deodorant agent, and a cosmetic for nail. Among these, make-up cosmetics such as milky lotion, cream, hair care, and foundation, and a cosmetic with an imparted sunscreen effect are particularly preferable. As a shape of the inventive cosmetic, various shapes such as liquid, cream, solid, paste, gel, mousse, souffle, cray, powder, and stick can be selected.

### EXAMPLE

Hereinafter, the present invention will be described more specifically with showing Examples and Comparative Examples, but the present invention is not limited to the following Examples.

### Example 1

Into a reaction vessel, 12.96 parts by mass of an organosiloxane represented by the following average composition formula (1-2), 66.04 parts by mass of an organosiloxane represented by the following average composition formula (2-2), 79.00 parts by mass of isopropyl alcohol, and 0.04 parts by mass of a 1 mass% ethanol solution of chloroplatinic acid as a catalyst for a hydrosilylation reaction were added. The mixture was reacted for 3 hours with keeping an internal temperature at 70 to 80°C, then heated to the internal temperature of 100°C under a reduced pressure, and a volatile component was removed to obtain a colorless, semitransparent, elastic, soft, resin-like phenyl-modified crosslinked organosiloxane. In this time, a ratio between the phenyl groups and the silicon atoms was 0.34.

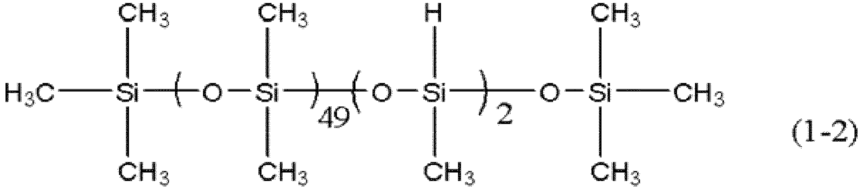

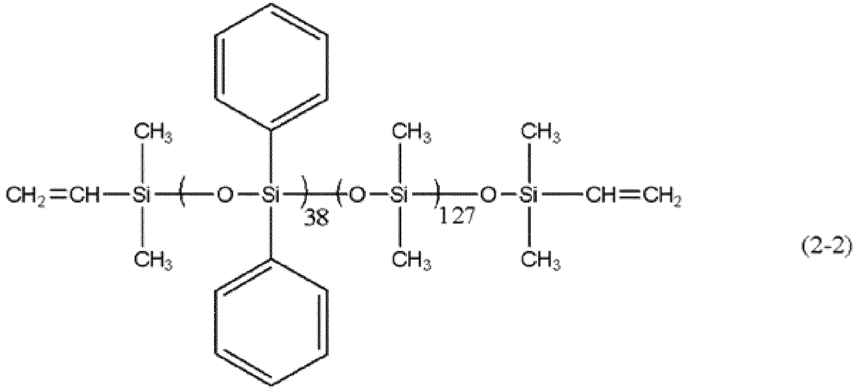

### Example 2

Mixing 20.00 g of the resin obtained in Example 1 and diphenylsiloxy phenyl trimethicone (labeled name) (KF-56A, manufactured by Shin-Etsu Chemical Co., Ltd.) was performed by using a triple-roller to obtain a paste composition at a concentration of 12%. In this time, the refractive index was 1.496, and the viscosity was 8295 mPa·s.

In the present Examples, the described viscosity was a measured value using MCR102, manufactured by Anton Paar GmbH with a shearing rate of 20 (1/s). The measurement conditions were at 25°C, 50-mm parallel plate, and 0.5-mm gap.

### Example 3

Into a reaction vessel, 7.46 parts by mass of an organosiloxane represented by the following average composition formula (1-3), 51.89 parts by mass of an organosiloxane represented by the average composition formula (2-2), 59.35 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, soft, resin-like phenyl-modified crosslinked organosiloxane. In this time, a ratio between the phenyl groups and the silicon atoms was 0.36.

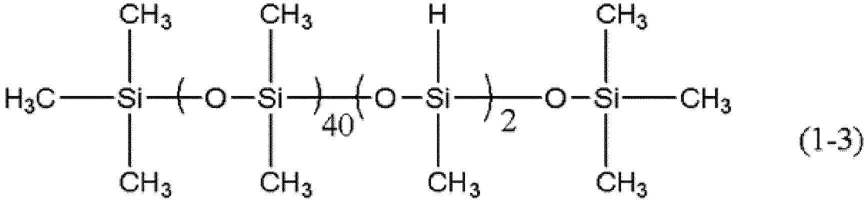

### Example 4

Mixing 20.00 g of the resin obtained in Example 3 and diphenylsiloxy phenyl trimethicone (labeled name) (KF-56A, manufactured by Shin-Etsu Chemical Co., Ltd.) was performed by using a triple-roller to obtain a paste composition at a concentration of 12%. In this time, the refractive index was 1.496, and the viscosity was 13382 mPa·s.

### Example 5

Into a reaction vessel, 48.70 parts by mass of an organosiloxane represented by the following average composition formula (1-4), 12.68 parts by mass of an organosiloxane represented by the following average composition formula (2-3), 61.38 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, soft, resin-like phenyl-modified crosslinked organosiloxane. In this time, a ratio between the phenyl groups and the silicon atoms was 0.37.

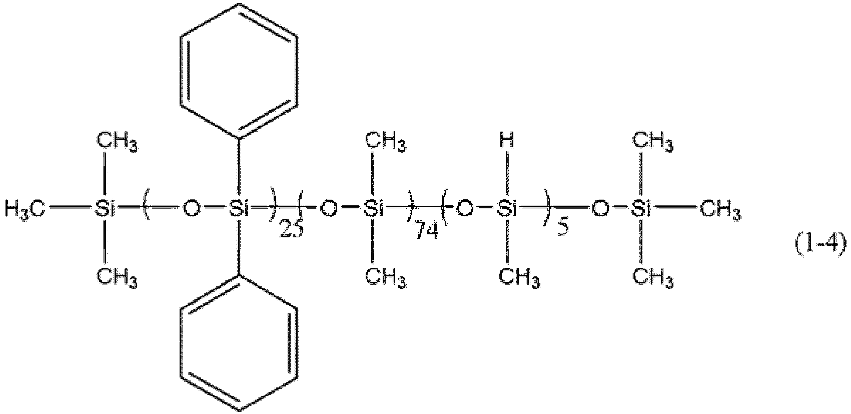

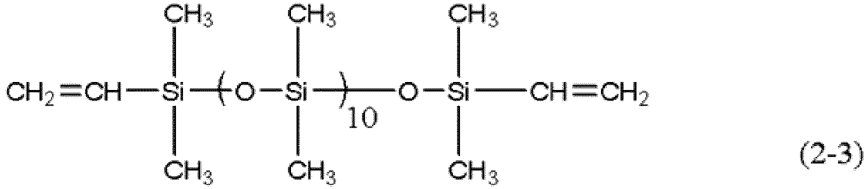

### Example 6

Mixing 20.00 g of the resin obtained in Example 5 and diphenylsiloxy phenyl trimethicone (labeled name) (KF-56A, manufactured by Shin-Etsu Chemical Co., Ltd.) was performed by using a triple-roller to obtain a paste composition at a concentration of 25%. In this time, the refractive index was 1.493, and the viscosity was 36672 mPa·s.

### Example 7

Into a reaction vessel, 1.66 parts by mass of an organosiloxane represented by the following average composition formula (1-5), 3.89 parts by mass of an organosiloxane represented by the following average composition formula (1-6), 55.94 parts by mass of an organosiloxane represented by the following average composition formula (2-4), 62.01 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, resin-like phenyl-modified crosslinked organosiloxane. In this time, a ratio between the phenyl groups and the silicon atoms was 0.44.

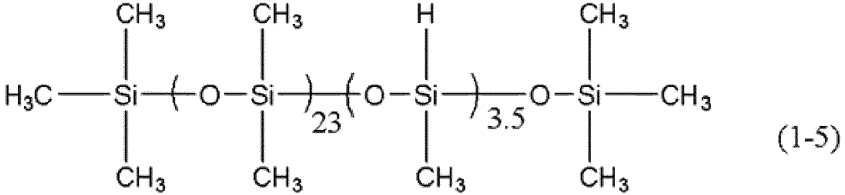

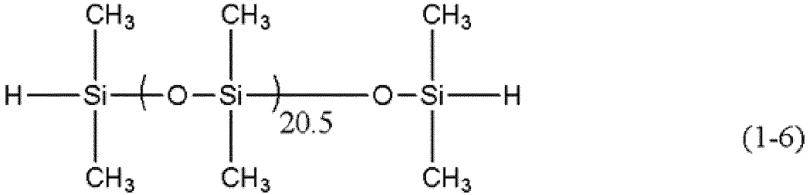

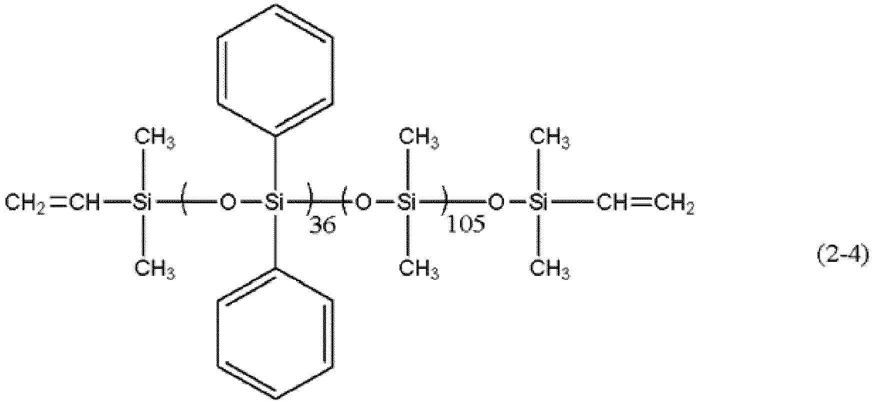

### Example 8

Mixing 20.00 g of the resin obtained in Example 7 and neopentyl glycol diethylhexanoate (labeled name) (COSMOL 525, manufactured by The Nisshin OilliO Group, Ltd.) was performed by using a triple-roller to obtain a paste composition at a concentration of 14%. In this time, the refractive index was 1.444, and the viscosity was 25253 mPa·s.

### Example 9

Into a reaction vessel, 5.11 parts by mass of an organosiloxane represented by the following average composition formula (1-7), 58.51 parts by mass of an organosiloxane represented by the following average composition formula (2-5), 63.61 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, resin-like phenyl-modified crosslinked organosiloxane. In this time, a ratio between the phenyl groups and the silicon atoms was 0.63.

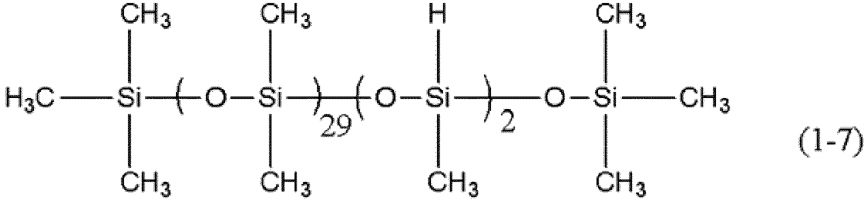

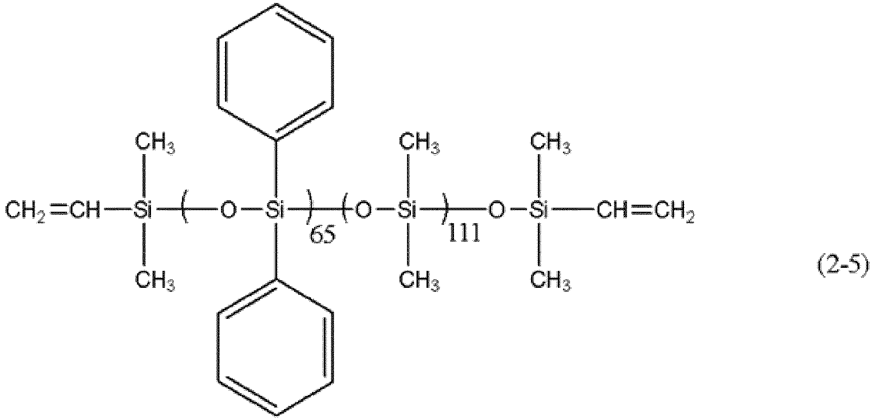

### Example 10

Mixing 20.00 g of the resin obtained in Example 9 and diphenylsiloxy phenyl trimethicone (labeled name) (KF-56A, manufactured by Shin-Etsu Chemical Co., Ltd.) was performed by using a triple-roller to obtain a paste composition at a concentration of 22%. In this time, the refractive index was 1.502, and the viscosity was 5612 mPa·s.

### Example 11

Into a reaction vessel, 8.18 parts by mass of an organosiloxane represented by the following average composition formula (1-8), 51.91 parts by mass of an organosiloxane represented by the following average composition formula (2-6), 61.38 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, resin-like phenyl-modified crosslinked organosiloxane. In this time, a ratio between the phenyl groups and the silicon atoms was 0.80.

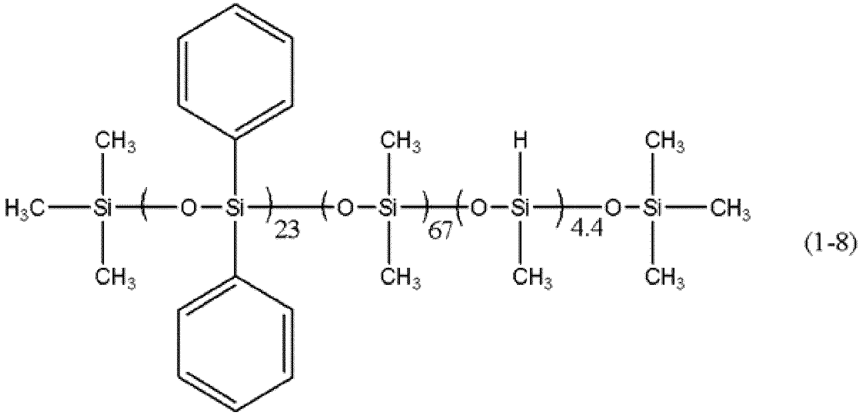

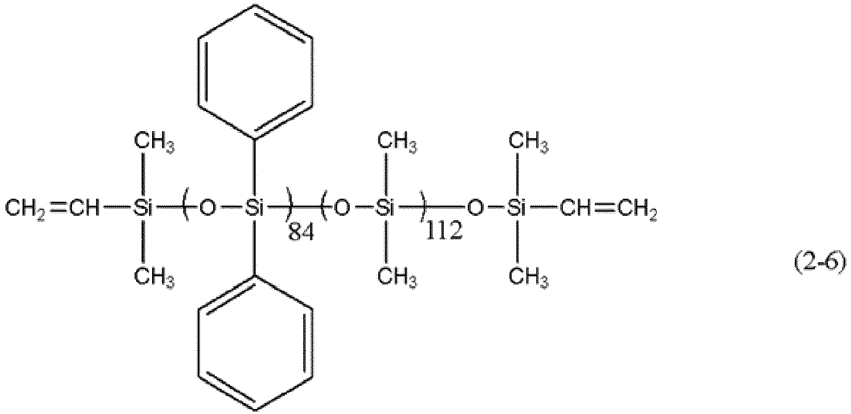

### Example 12

Mixing 20.00 g of the resin obtained in Example 11 and diphenylsiloxy phenyl trimethicone (labeled name) (KF-56A, manufactured by Shin-Etsu Chemical Co., Ltd.) was performed by using a triple-roller to obtain a paste composition at a concentration of 30%. In this time, the refractive index was 1.508, and the viscosity was 9797 mPa·s.

### Example 13

Into a reaction vessel, 54.33 parts by mass of an organosiloxane represented by the following average composition formula (1-9), 61.32 parts by mass of an organosiloxane represented by the average composition formula (2-2), and 0.06 parts by mass of a 1 mass% ethanol solution of chloroplatinic acid were added. The mixture was reacted for 3 hours with keeping an internal temperature at 70 to 80°C, and then a white, viscous phenyl-modified crosslinked organosiloxane was obtained. In this time, a ratio between the phenyl groups and the silicon atoms was 0.37.

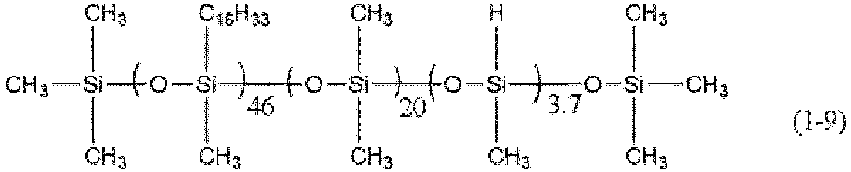

### Example 14

Mixing 20.00 g of the resin obtained in Example 13 and isododecane (labeled name) (MARUKASOL R, manufactured by Maruzen Petrochemical Co., Ltd.) was performed by using a Disper to obtain a paste composition at a concentration of 90%. In this time, the refractive index was 1.473, and the viscosity was 15427 mPa·s.

### Example 15

Into a reaction vessel, 3.75 parts by mass of an organosiloxane represented by the average composition formula (1-5), 1.46 parts by mass of an organosiloxane represented by the following average composition formula (2-7), 49.53 parts by mass of an organosiloxane represented by the average composition formula (2-2), 55.78 parts by mass of isopropyl alcohol, and 0.02 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a colorless, semitransparent, elastic, resin-like phenyl-modified crosslinked organosiloxane. In this time, a ratio between the phenyl groups and the silicon atoms was 0.39.

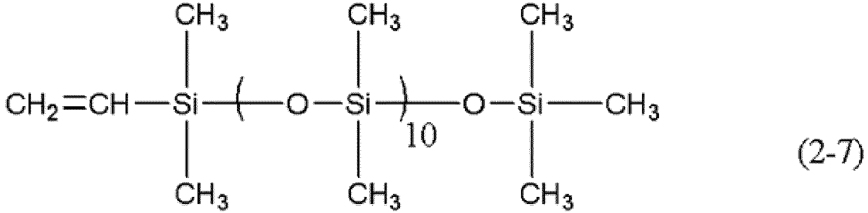

### Example 16

Mixing 20.00 g of the resin obtained in Example 15 and diphenylsiloxy phenyl trimethicone (labeled name) (KF-56A, manufactured by Shin-Etsu Chemical Co., Ltd.) was performed by using a triple-roller to obtain a paste composition at a concentration of 16%. In this time, the refractive index was 1.495, and the viscosity was 19609 mPa·s.

### Example 17

Into a reaction vessel, 8.03 parts by mass of an organosiloxane represented by the following average composition formula (1-10), 38.20 parts by mass of an organosiloxane represented by the following average composition formula (2-8), 46.23 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a white, soft, amorphous, resin-like phenyl-modified crosslinked organosiloxane. In this time, a ratio between the phenyl groups and the silicon atoms was 0.36.

[(CH₃)₃SiO₁/₂]_{15.8}[(CH₃)₂HSiO₁/₂]_{3.5}[SiO₂]_{15.3} (1-10)

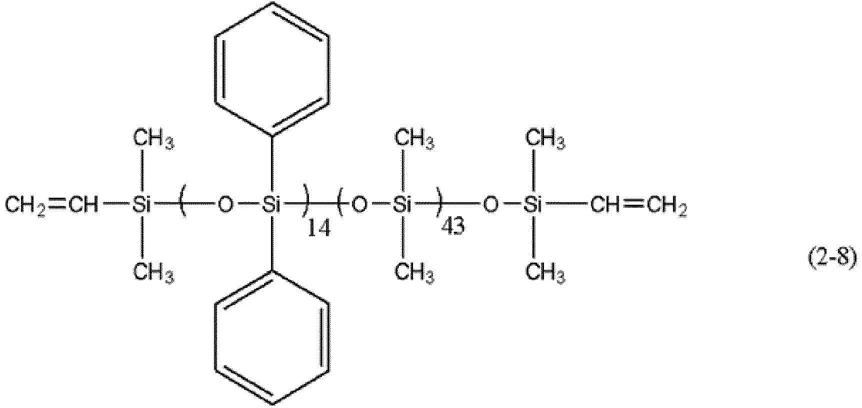

### Example 18

Mixing 20.00 g of the resin obtained in Example 17 and diphenylsiloxy phenyl trimethicone (labeled name) (KF-56A, manufactured by Shin-Etsu Chemical Co., Ltd.) was performed by using a triple-roller to obtain a paste composition at a concentration of 15%. In this time, the refractive index was 1.495, and the viscosity was 16437 mPa·s.

### Example 19

Into a reaction vessel, 4.09 parts by mass of an organosiloxane represented by the average composition formula (1-7), 32.77 parts by mass of an organosiloxane represented by the average composition formula (2-2), 84.00 parts by mass of diphenylsiloxy phenyl trimethicone (labeled name) (KF-56A, manufactured by Shin-Etsu Chemical Co., Ltd.), and 0.03 parts by mass of a 1 mass% divinyltetramethyldisiloxane solution of chloroplatinic acid were added. The mixture was reacted for 3 hours with keeping an internal temperature at 70 to 80°C, and then mixed with 304.9 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.497, and the viscosity was 116640 mPa·s. A ratio between the phenyl groups and the silicon atoms was 0.37.

### Example 20

Into a reaction vessel, 2.56 parts by mass of an organosiloxane represented by the average composition formula (1-7), 27.92 parts by mass of an organosiloxane represented by the following average composition formula (2-9), 71.11 parts by mass of diphenylsiloxy phenyl trimethicone (labeled name) (KF-56A, manufactured by Shin-Etsu Chemical Co., Ltd.), and 0.02 parts by mass of a 1 mass% divinyltetramethyldisiloxane solution of chloroplatinic acid were added. The mixture was reacted for 3 hours with keeping an internal temperature at 70 to 80°C, and then mixed with 256.90 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.499, and the viscosity was 6370 mPa·s. A ratio between the phenyl groups and the silicon atoms was 0.50.

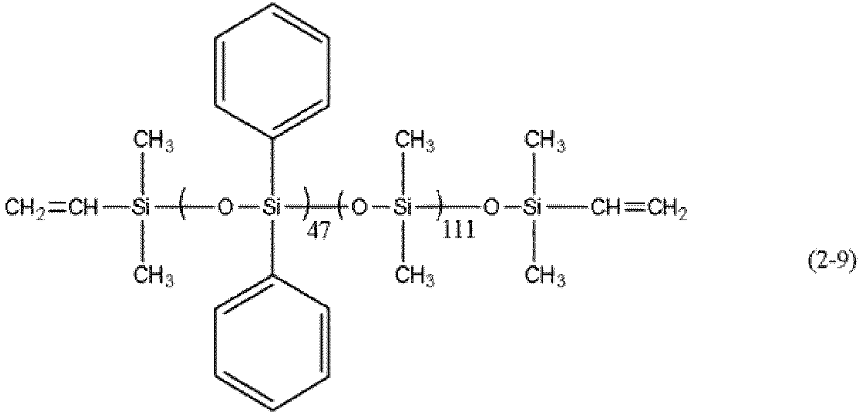

### Example 21

Into a reaction vessel, 2.37 parts by mass of an organosiloxane represented by the following average composition formula (1-11), 30.58 parts by mass of an organosiloxane represented by the average composition formula (2-9), 76.88 parts by mass of diphenylsiloxy phenyl trimethicone (labeled name) (KF-56A, manufactured by Shin-Etsu Chemical Co., Ltd.), and 0.02 parts by mass of a 1 mass% divinyltetramethyldisiloxane solution of chloroplatinic acid were added. The mixture was reacted for 3 hours with keeping an internal temperature at 70 to 80°C, and then mixed with 462.15 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.499, and the viscosity was 12500 mPa.s. A ratio between the phenyl groups and the silicon atoms was 0.51.

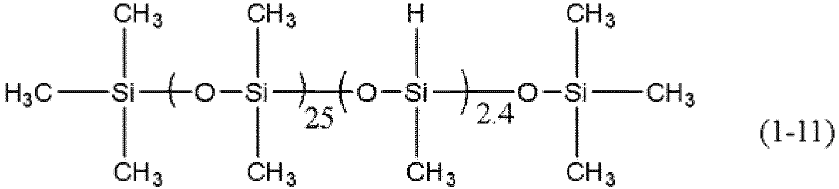

### Example 22

Into a reaction vessel, 2.86 parts by mass of an organosiloxane represented by the average composition formula (1-7), 32.77 parts by mass of an organosiloxane represented by the average composition formula (2-5), 84.00 parts by mass of diphenylsiloxy phenyl trimethicone (labeled name) (KF-56A, manufactured by Shin-Etsu Chemical Co., Ltd.), and 0.02 parts by mass of a 1 mass% ethanol solution of chloroplatinic acid were added. The mixture was reacted for 3 hours with keeping an internal temperature at 70 to 80°C, and then mixed with 299.5 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.499, and the viscosity was 3990 mPa·s. A ratio between the phenyl groups and the silicon atoms was 0.63.

### Example 23

Into a reaction vessel, 4.00 parts by mass of an organosiloxane represented by the following average composition formula (1-12), 40.09 parts by mass of an organosiloxane represented by the average composition formula (2-2), 66.14 parts by mass of diphenylsiloxy phenyl trimethicone (labeled name) (KF-56A, manufactured by Shin-Etsu Chemical Co., Ltd.), and 0.02 parts by mass of a 1 mass% ethanol solution of chloroplatinic acid were added. The mixture was reacted for 3 hours with keeping an internal temperature at 70 to 80°C, and then mixed with 408.5 parts by mass of diphenylsiloxy phenyl trimethicone (labeled name) (KF-56A, manufactured by Shin-Etsu Chemical Co., Ltd.) by using a triple-roller to be uniform to obtain a paste composition at a concentration of 11%. In this time, the refractive index was 1.497, and the viscosity was 22430 mPa·s. A ratio between the phenyl groups and the silicon atoms was 0.39.

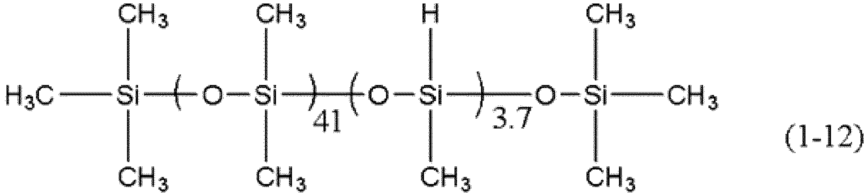

### Example 24

Into a reaction vessel, 4.36 parts by mass of an organosiloxane represented by the following average composition formula (1-13), 29.40 parts by mass of an organosiloxane represented by the average composition formula (2-9), 78.76 parts by mass of diphenylsiloxy phenyl trimethicone (labeled name) (KF-56A, manufactured by Shin-Etsu Chemical Co., Ltd.), and 0.02 parts by mass of a 1 mass% ethanol solution of chloroplatinic acid were added. The mixture was reacted for 3 hours with keeping an internal temperature at 70 to 80°C, and then mixed with 284.66 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.498, and the viscosity was 10170 mPa·s. A ratio between the phenyl groups and the silicon atoms was 0.46.

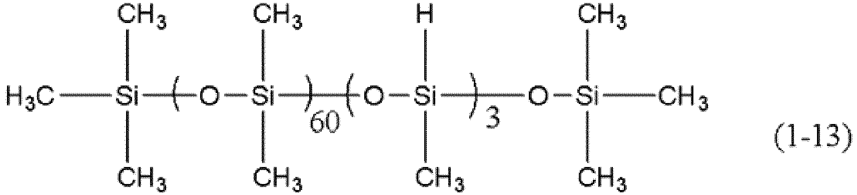

### Comparative Example 1

Into a reaction vessel, 49.73 parts by mass of an organosiloxane represented by the average composition formula (1-9), 54.09 parts by mass of an organosiloxane represented by the following average composition formula (2-10), 44.50 parts by mass of isopropyl alcohol, and 0.05 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a brown, semitransparent, viscous phenyl-modified crosslinked organosiloxane. In this time, a ratio between the phenyl groups and the silicon atoms was 0.18.

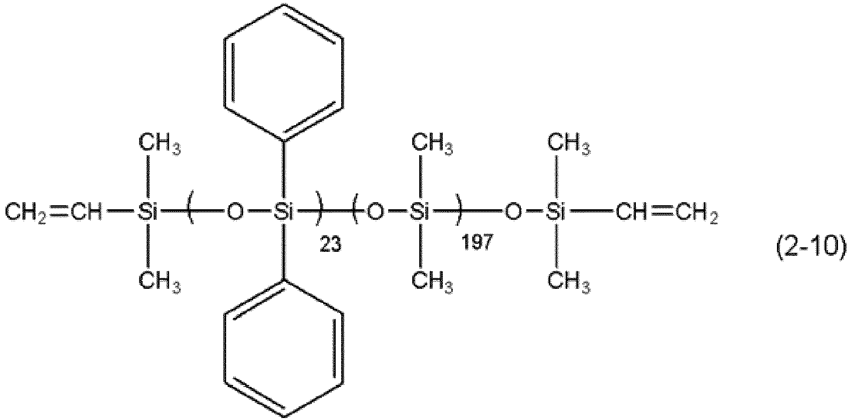

### Comparative Example 2

Mixing 20.00 g of the resin obtained in Comparative Example 1 and isododecane (labeled name) (MARUKASOL R, manufactured by Maruzen Petrochemical Co., Ltd.) was performed by using a Disper to be uniform to obtain a paste composition at a concentration of 70%. In this time, the refractive index was 1.49, and the viscosity was 8828 mPa·s.

### Comparative Example 3

Into a reaction vessel, 13.55 parts by mass of an organosiloxane represented by the following average composition formula (1-14), 45.28 parts by mass of an organosiloxane represented by the average composition formula (2-2), 59.23 parts by mass of isopropyl alcohol, and 0.03 parts by mass of a 1 mass% ethanol solution of chloroplatinic acid were added. The mixture was reacted for 3 hours with keeping an internal temperature at 70 to 80°C, then heated to the internal temperature of 100°C under a reduced pressure, and a volatile component was removed to obtain a brown, semitransparent, viscous phenyl-modified crosslinked organosiloxane. In this time, a ratio between the phenyl groups and the silicon atoms was 0.29.

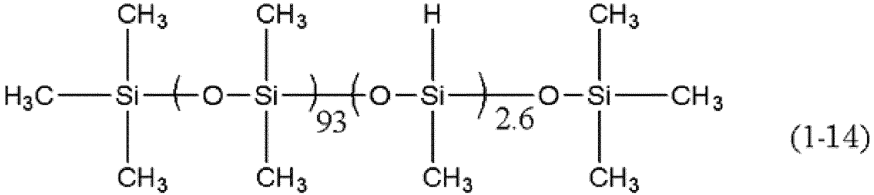

### Comparative Example 4

Mixing 20.00 g of the resin obtained in Comparative Example 3 and diphenylsiloxy phenyl trimethicone (labeled name) (KF-56A, manufactured by Shin-Etsu Chemical Co., Ltd.) was performed by using a triple-roller to obtain a paste composition at a concentration of 16%. In this time, the refractive index was 1.49, and the viscosity was 38013 mPa·s.

### Comparative Example 5

Into a reaction vessel, 3.61 parts by mass of an organosiloxane represented by the following average composition formula (1-14), 12.08 parts by mass of an organosiloxane represented by the average composition formula (2-2), 88.90 parts by mass of diphenylsiloxy phenyl trimethicone (labeled name) (KF-56A, manufactured by Shin-Etsu Chemical Co., Ltd.), and 0.01 parts by mass of a 1 mass% ethanol solution of chloroplatinic acid were added. The mixture was reacted for 3 hours with keeping an internal temperature at 70 to 80°C, and then mixed with 80.00 parts by mass of diphenylsiloxy phenyl trimethicone (labeled name) (KF-56A, manufactured by Shin-Etsu Chemical Co., Ltd.) to be uniform to obtain a paste composition at a concentration of 8.5%. In this time, the refractive index was 1.46, and the viscosity was 5420 mPa.s. A ratio between the phenyl groups and the silicon atoms was 0.29.

Table 1 shows: the ratios between the phenyl groups and the silicon atoms (hereinafter, described as "phenyl amount") determined by calculation with the average composition formulae of the phenyl-modified crosslinked organosiloxanes obtained in Examples 1, 3, 5, 7, 9, 11, 13, 15, and 17 and Comparative Examples 1 and 3; and absorbability of each oil agent.

The absorbability was evaluated as follows. The phenyl-modified crosslinked organosiloxane and an oil agent at the same amount were mixed and shaken with a shaker for 12 hours, and then the appearance was visually observed to evaluate the result as follows.
Excellent: Excellent absorption was observed.
Good: Absorption was observed.
Fair: A small amount of the oil agent not absorbed was observed.
Poor: Almost all the oil agent was not absorbed.

**[Table 1]**

| | Ex. 1 | Ex. 3 | Ex. 5 | Ex. 7 | Ex. 9 | Ex. 11 |
|---|---|---|---|---|---|---|
| Phenyl amount | 0.34 | 0.36 | 0.37 | 0.44 | 0.63 | 0.80 |
| Ethylhexyl methoxycinnamate | Good | Good | Good | Excellent | Excellent | Excellent |
| Diphenylsiloxy phenyl trimethicone (NOTE 1) | Excellent | Excellent | Excellent | Excellent | Excellent | Excellent |
| Neopentyl glycol diethylhexanoate | Excellent | Excellent | Excellent | Excellent | Excellent | Excellent |
| Isododecane | Excellent | Excellent | Excellent | Excellent | Good | Poor |
| Dimethicone (6cs) (NOTE 2) | Good | Fair | Good | Poor | Poor | Poor |
| Dimethicone (2cs) (NOTE 3) | Excellent | Excellent | Excellent | Excellent | Poor | Poor |
| Cyclopentasiloxane (NOTE 4) | Excellent | Excellent | Excellent | Excellent | Poor | Poor |

| | Ex. 13 | Ex. 15 | Ex. 17 | Comp. Ex. 1 | Comp. Ex. 3 | |
|---|---|---|---|---|---|---|
| Phenyl amount | 0.37 | 0.39 | 0.36 | 0.18 | 0.29 | |
| Ethylhexyl methoxycinnamate | Good | Good | Good | Poor | Good | |
| Diphenylsiloxy phenyl trimethicone (NOTE 1) | Good | Excellent | Excellent | Good | Excellent | |
| Neopentyl glycol diethylhexanoate | Good | Excellent | Excellent | Excellent | Excellent | |
| Isododecane | Good | Excellent | Excellent | Excellent | Excellent | |
| Dimethicone (6cs) (NOTE 2) | Poor | Good | Fair | Good | Excellent | |
| Dimethicone (2cs) (NOTE 3) | Good | Good | Excellent | Good | Excellent | |
| Cyclopentasiloxane (NOTE 4) | Good | Good | Excellent | Good | Excellent | |

| | | | | | | |
|---|---|---|---|---|---|---|
| (NOTE 1) KF-56A, manufactured by Shin-Etsu Chemical Co., Ltd. (NOTE 2) KF-96A-6CS, manufactured by Shin-Etsu Chemical Co., Ltd. (NOTE 3) KF-96L-2CS, manufactured by Shin-Etsu Chemical Co., Ltd. (NOTE 4) KF-995, manufactured by Shin-Etsu Chemical Co., Ltd. | | | | | | |

From Table 1, a phenyl amount of 0.29 or more exhibited good absorbability of ethylhexyl methoxycinnamate and phenylmethylsilicone oil, which were polar oils. Meanwhile, Comparative Example 1 had a phenyl group of less than 0.34, and absorbed almost no ethylhexyl methoxycinnamate. Comparative Example 3 exhibited good absorbability of all the oil agents, and remarkably excellent absorbability of dimethicone, which indicated good compatibility with a silicone oil.

Table 2 shows results of a HAZE value of a mixture of 5 g of the paste composition and 2 g of the oil agent used for dilution in producing the composition subtracted from a HAZE value of a mixture of 5 g of the paste composition and 2 g of ethylhexyl methoxycinnamate. The paste composition was obtained by mixing the oil agent and the phenyl-modified crosslinked organosiloxane obtained in Examples 2, 4, 6, 8, 10, 12, 14, 16, 18, and 19 to 24 and Comparative Examples 2, 4, and 5. Table 2 also shows a result of a similar evaluation performed by using a paste composition of 15% of KSG-18A (diphenylsiloxy phenyl trimethicone (labeled name) manufactured by Shin-Etsu Chemical Co., Ltd., which was a phenyl-modified crosslinked silicone available as a commercial product, and 85% of diphenylsiloxy phenyl trimethicone.

The HAZE value was measured by sandwiching the sample with 1.1 mm in thickness by glass plates with 76 mm × 52 mm. For the measurement, HAZE METER NDH 5000SP, manufactured by NIPPON DENSHOKU INDUSTRIES CO., LTD., was used.

The value of the HAZE value of the mixture with the oil agent for dilution subtracted from the HAZE value of the mixture with ethylhexyl methoxycinnamate are described as the following criteria.
Excellent: less than 0
Good: 0 or more and less than 10
Fair: 10 or more and less than 40
Poor: 40 or more

**[Table 2]**

| | Ex. 2 | Ex. 4 | Ex. 6 | Ex. 8 | Ex. 10 | Ex. 12 | Ex. 14 | Ex. 16 | Ex. 18 | Ex. 19 |
|---|---|---|---|---|---|---|---|---|---|---|
| Phenyl amount | 0.34 | 0.36 | 0.37 | 0.44 | 0.63 | 0.80 | 0.37 | 0.39 | 0.36 | 0.37 |
| Measurement result | Good | Good | Fair | Excellent | Excellent | Good | Good | Good | Fair | Good |

| | Ex. 20 | Ex. 21 | Ex. 22 | Ex. 23 | Ex. 24 | Comp. Ex. 2 | Comp. Ex. 4 | Comp. Ex. 5 | KSG-18A | |
|---|---|---|---|---|---|---|---|---|---|---|
| Phenyl amount | 0.5 | 0.51 | 0.63 | 0.39 | 0.46 | 0.18 | 0.29 | 0.29 | - | |
| Measurement result | Good | Good | Good | Fair | Fair | Poor | Poor | Good | Poor | |

From Table 2, no large difference of the HAZE values between the case of mixing with ethylhexyl methoxycinnamate and the case of mixing the above dilution oil agent was observed in Examples, and the appearance was not impaired. Comparative Example 2, which used the resin almost not absorbing ethylhexyl methoxycinnamate (Comparative Example 1), considerably impaired the appearance from the evaluation result of the HAZE value. Comparative Example 4 exhibited an oil-absorbing property of ethylhexyl methoxycinnamate with the resin itself (Comparative Example 3), but largely changed the HAZE value and considerably impaired the appearance. Comparative Example 5, which had a lower concentration than Comparative Example 4, yielded no large difference in the HAZE value, and this was presumably because of the low concentration. With KSG-18A, which was a commercial product, the impaired appearance was also observed. Although the resin obtained by extracting KSG-18A with hexane failed to absorb the same amount of ethylhexyl methoxycinnamate, the resin absorbed the same amount of dimethylsilicone (6cS and 2cS) and cyclopentasiloxane. Thus, the phenyl amount of the resin was judged to be less than 0.29.

### Examples 25 to 28 and Comparative Example 6

W/O creams were produced with preparations shown in Table 3.

**[Table 3]**

| Component | | Blank | Example | | | | Comparative Example |
|---|---|---|---|---|---|---|---|
| | | 1 | 25 | 26 | 27 | 28 | 6 |
| 1 | Composition of Ex. 19 (8.5%) | | 3.5 | | | | |
| | Composition of Ex. 20 (8.5%) | | | 3.5 | | | |
| | Composition of Ex. 21 (8.5%) | | | | 3.5 | | |
| | Composition of Ex. 22 (8.5%) | | | | | 3.5 | |
| | KSG-18A (NOTE 1) | | | | | | 2 |
| 2 | KF-6048 (NOTE 2) | 2 | 2 | 2 | 2 | 2 | 2 |
| 3 | KF-56A (NOTE 3) | Rest | Rest | Rest | Rest | Rest | Rest |
| 4 | BG | 5 | 5 | 5 | 5 | 5 | 5 |
| 5 | Na chloride | 0.5 | 0.5 | 0.5 | 0.5 | 0.5 | 0.5 |
| 6 | Water | 64.5 | 64.5 | 64.5 | 64.5 | 64.5 | 64.5 |
| Total | | 100 | 100 | 100 | 100 | 100 | 100 |
| Amount of crosslinked phenyl-modified silicone | | 0. 00 | 0.30 | 0.30 | 0.30 | 0.30 | 0.30 |

| | | | | | | | |
|---|---|---|---|---|---|---|---|
| (NOTE 1) Mixture of (diphenylsiloxy phenyl trimethicone (:labeled name) 85% + (dimethicone / phenyl vinyl dimethicone) crosspolymer (:labeled name) 15%, manufactured by Shin-Etsu Chemical Co., Ltd. (NOTE 2) Cetyl PEG/PPG-10/1 dimethicone (:labeled name), manufactured by Shin-Etsu Chemical Co., Ltd. (NOTE 3) Diphenylsiloxy phenyl trimethicone (:labeled name), manufactured by Shin-Etsu Chemical Co., Ltd. | | | | | | | |

### (Manufacturing Method)

The components 1 to 3 were mixed to be uniform. After the mixing to be uniform the components 4 to 6, it was gently added therein to form an emulsion. This was loaded into a predetermined container to obtain a milky lotion.

Of the obtained W/O milky lotion, (1) feel of use (absence of stickiness), (2) coatability (spreading and staying properties of the cosmetic), and (3) a thickening property were evaluated with the following criteria. Table 4 shows the evaluation results of Examples 25 to 28 and Comparative Example 6.

### Characteristics Evaluation

Of the cosmetics, items of the feel of use and coatability were evaluated by 10 professional panelists with the evaluation criteria described below. Based on the average value by the 10 panelists, the results were judged in accordance with the judgement criteria.

### Evaluation Criteria

5 points: Excellent
4 points: Good
3 points: Average
2 points: Fair
1 point: Poor

The average point obtained as above was judged in accordance with the following criteria.

### Judgement of Average Point:

The obtained average point was 4.5 or more:
   Excellent
The obtained average point was 3.5 or more and less than 4.5: Good
The obtained average point was 2.5 or more and less than 3.5: Fair
The obtained average point was 1.5 or more and less than 2.5: Poor
The obtained average point was less than 1.5:
   Bad

The judgement of "Good" or higher was evaluated as being passed.

### Thickening Property

The thickening property was evaluated with a thickening ratio compared with a blank in which the crosslinked phenyl-modified silicone mixture was not blended. The viscosity was measured by using a B-type viscosimeter (TVB-10 model, manufactured by Toki Sangyo Co., Ltd.), with a spindle No. 4 at 6 rpm for a measurement time of 60 seconds.
Excellent: 200% or more
Good: 150% or more and less than 175%
Fair: 125% or more and less than 150%
Poor: 100% or more and less than 125%
Bad: less than 100%

The judgement of "Good" or higher was evaluated as being passed.

**[Table 4]**

| | | Blank | Example | | | | Comparative Example |
|---|---|---|---|---|---|---|---|
| No. | Evaluation item | 1 | 25 | 26 | 27 | 28 | 6 |
| (1) | Feel of use | Poor | Good | Excellent | Good | Good | Fair |
| (2) | Coatability | Fair | Excellent | Excellent | Good | Excellent | Good |
| (3) | Thickening property | - | Excellent | Excellent | Good | Good | Bad |

As obvious from Table 4, the W/O milky lotions of Examples 25 to 28 were demonstrated to have good feel of use, coatability, and thickening property compared with Comparative Example 6.

### Examples 29 to 32 and Comparative Examples 7 to 9

W/O creams were produced with the preparations shown in Table 5.

**[Table 5]**

| Component | | Blank | Example | | | | Comparative Example | | |
|---|---|---|---|---|---|---|---|---|---|
| | | 2 | 29 | 30 | 31 | 32 | 7 | 8 | 9 |
| 1 | Composition of Ex. 19 (8.5%) | | 3 | | | | | | |
| | Composition of Ex. 20 (8.5%) | | | 3 | | | | | |
| | Composition of Ex. 21 (8.5%) | | | | 3 | | | | |
| | Composition of Ex. 22 (8.5%) | | | | | 3 | | | |
| | KSG-18A (NOTE 1) | | | | | | 1.7 | 3 | |
| | Comp. Ex. 5 (8.5%) | | | | | | | | 3 |
| 2 | KSG-240 (NOTE 2) | 3.5 | 3.5 | 3.5 | 3.5 | 3.5 | 3.5 | 3.5 | 3.5 |
| 3 | KF-6048 (NOTE 3) | 1.5 | 1.5 | 1.5 | 1.5 | 1.5 | 1.5 | 1.5 | 1.5 |
| 4 | Ethylhexyl methoxycinnamate | 9.5 | 9.5 | 9.5 | 9.5 | 9.5 | 9.5 | 9.5 | 9.5 |
| 5 | Octocrylene | 2.5 | 2.5 | 2.5 | 2.5 | 2.5 | 2.5 | 2.5 | 2.5 |
| 6 | Hexyl diethylaminohydroxybenzoylbenzoate | 3 | 3 | 3 | 3 | 3 | 3 | 3 | 3 |
| 7 | Ethylhexyl salicylate | 5 | 5 | 5 | 5 | 5 | 5 | 5 | 5 |
| 8 | KF-995 (NOTE 4) | 2 | 2 | 2 | 2 | 2 | 2 | 2 | 2 |
| 9 | KF-56A (NOTE 5) | Rest | Rest | Rest | Rest | Rest | Rest | Rest | Rest |
| 10 | BG | 3 | 3 | 3 | 3 | 3 | 3 | 3 | 3 |
| 11 | Na citrate | 0.5 | 0.5 | 0.5 | 0.5 | 0.5 | 0.5 | 0.5 | 0.5 |
| 12 | Na chloride | 0.5 | 0.5 | 0.5 | 0.5 | 0.5 | 0.5 | 0.5 | 0.5 |
| 13 | Water | 61 | 61 | 61 | 61 | 61 | 61 | 61 | 61 |
| Total | | 100 | 100 | 100 | 100 | 100 | 100 | 100 | 100 |
| Amount of crosslinked phenyl-modified silicone | | 0 | 0.26 | 0.26 | 0.26 | 0.26 | 0.26 | 0.45 | 0.26 |

| | | | | | | | | | |
|---|---|---|---|---|---|---|---|---|---|
| (NOTE 1) Mixture of diphenylsiloxy phenyl trimethicone (:labeled name) 85% + (dimethicone / phenyl vinyl methicone) crosspolymer (:labeled name) 15%, manufactured by Shin-Etsu Chemical Co., Ltd. (NOTE 2) Mixture of (dimethicone / (PEG-10/15)) crosspolymer (:labeled name) 20% and cyclopentasiloxane (:labeled name) 80% (NOTE 3) Cetyl PEG/PPG-10/1 dimethicone (:labeled name), manufactured by Shin-Etsu Chemical Co., Ltd. (NOTE 4) Cyclopentasiloxane (:labeled name), manufactured by Shin-Etsu Chemical Co., Ltd. (NOTE 5) Diphenylsiloxy phenyl trimethicone (:labeled name), manufactured by Shin-Etsu Chemical Co., Ltd. | | | | | | | | | |

### (Manufacturing Method)

The components 1 to 9 were mixed to be uniform. To this, the components 10 to 13 after the mixing to be uniform, was gently added to form an emulsion. This was loaded into a predetermined container to obtain a sunscreen cream milky lotion.

Of the obtained W/O sunscreen cream, (1) feel of use (absence of stickiness), (2) coatability (spreading and staying properties of the cosmetic), (3) a thickening property, and (4) stability were evaluated with the following criteria. Table 6 shows the evaluation results of Examples 29 to 32 and Comparative Examples 7 to 9.

### Characteristics Evaluation

Of the cosmetics, items of the feel of use and coatability were evaluated by 10 professional panelists with the evaluation criteria described below. Based on the average value by the 10 panelists, the results were judged in accordance with the judgement criteria.

### Evaluation Criteria

5 points: Excellent
4 points: Good
3 points: Average
2 points: Fair
1 point: Poor

The average point obtained as above was judged in accordance with the following criteria.

### Judgement of Average Point:

The obtained average point was 4.5 or more:
   Excellent
The obtained average point was 3.5 or more and less than 4.5: Good
The obtained average point was 2.5 or more and less than 3.5: Fair
The obtained average point was 1.5 or more and less than 2.5: Poor
The obtained average point was less than 1.5:
   Bad

The judgement of "Good" or higher was evaluated as being passed.

### Thickening Property

The thickening property was evaluated with a thickening ratio compared with a blank in which the crosslinked phenyl-modified silicone mixture was not blended. The viscosity was measured by using a B-type viscosimeter (TVB-10 model, manufactured by Toki Sangyo Co., Ltd.), with a spindle No. 4 at 6 rpm for a measurement time of 60 seconds.
Excellent: 120% or more
Good: 100% or more and less than 120%
Fair: 80% or more and less than 100%
Poor: 60% or more and less than 80%
Bad: less than 60%

The judgement of "Good" or higher was evaluated as being passed.

### Stability

The cosmetic was left to stand in a thermostat chamber at 50°C or -5°C, and a problem of appearance such as oil floating was checked.
Excellent: No problem occurred after one month at both of 50°C and -5°C.
Good: A problem occurred after one month at 50°C or -5°C.
Fair: A problem occurred after three weeks at 50°C or - 5°C.
Poor: A problem occurred after two weeks at 50°C or -5°C.
Bad: A problem occurred after one week at 50°C or -5°C.

The judgement of "Good" or higher was evaluated as being passed.

**[Table 6]**

| | | Blank | Example | | | | Comparative Example | | |
|---|---|---|---|---|---|---|---|---|---|
| No. | Evaluation item | 2 | 29 | 30 | 31 | 32 | 7 | 8 | 9 |
| (1) | Feel of use | Poor | Good | Excellent | Good | Good | Fair | Good | Good |
| (2) | Coatability | Poor | Excellent | Excellent | Good | Excellent | Fair | Good | Good |
| (3) | Thickening property | - | Good | Excellent | Excellent | Excellent | Bad | Fair | Fair |
| (4) | Stability | Bad | Good | Excellent | Excellent | Excellent | Poor | Poor | Poor |

As obvious from Table 6, the W/O milky lotions of Examples 29 to 32 were demonstrated to have good feel of use, coatability, thickening property, and stability compared with Comparative Examples 7 to 9. As found from the results of Comparative Examples 7 and 8, the present invention was found to be able to achieve stabilization that was not able to be achieved by thickening with increasing a blending amount of a simple crosslinked phenyl-modified silicone. Although Comparative Example 9 had a small change in the HAZE value as Comparative Example 5, the ratio between phenyl groups and silicon atoms was smaller than 0.34, and thereby the feel of use, coatability, thickening property, and stability were not good.

### Example 33

### W/O Sunscreen Cream

### <Preparation of Cosmetic>

A: The components 9 to 11 were uniformly dispersed with a Disper, and then the components 1 to 8 were added and mixed to be uniform;
B: The components 12 to 17 were mixed to be uniform; and
C: "B" was added into "A", and the mixture was emulsified to obtain a W/O sunscreen cream.

| Component | | mass% |
|---|---|---|
| 1. | KSG-320 (NOTE 1) | 3 |
| 2. | Composition of Example 23 (11%) | 2 |
| 3. | KF-6048 (NOTE 2) | 2.5 |
| 4. | Disteardimonium hectorite | 0.3 |
| 5. | Ethylhexyl methoxycinnamate | 7.5 |
| 6. | Octocrylene | 3 |
| 7. | Homosalate | 5 |
| 8. | Dicaprylyl carbonate | 7 |
| 9. | TMF-1.5 (NOTE 3) | 10.5 |
| 10. | KF-6105 (NOTE 4) | 1.5 |
| 11. | Alkylsilane-treated zinc oxide fine particle (NOTE 5) | 18 |
| 12. | BG | 3 |
| 13. | Ethanol | 5 |
| 14. | Sodium citrate | 0.2 |
| 15. | Sodium chloride | 1 |
| 16. | Allantoin | 0.2 |
| 17. | Purified water | Rest |
| Total | | 100 |

| | | |
|---|---|---|
| (NOTE 1) Mixture of (PEG-15 / lauryl dimethicone) crosspolymer (:labeled name) 25% and isododecane (:labeled name) 75%, manufactured by Shin-Etsu Chemical Co., Ltd. (NOTE 2) Cetyl PEG/PPG-10/1 dimethicone (:labeled name), manufactured by Shin-Etsu Chemical Co., Ltd. (NOTE 3) Methyl trimethicone (labeled name), manufactured by Shin-Etsu Chemical Co., Ltd. (NOTE 4) Lauryl polyglyceryl-3 polydimethyl siloxyethyl dimethicone (:labeled name), manufactured by Shin-Etsu Chemical Co., Ltd. (NOTE 5) AES-3083 treated, manufactured by Shin-Etsu Chemical Co., Ltd. | | |

The obtained W/O sunscreen cream had good feel of use and coatability, and excellent storage stability.

### Example 34

### W/O Shaking Sunscreen Cream

### <Preparation of Cosmetic>

A: The components 5 to 10 and 11 to 14 were dissolved with heating, and then mixed to be uniform with the components 1 to 4;
B: The components 15 to 19 were mixed to be uniform; and
C: "B" was added into "A", and the mixture was emulsified to obtain a W/O shaking sunscreen cream.

| Component | | mass% |
|---|---|---|
| 1. | Composition of Example 22 (8.5%) | 2 |
| 2. | KF-6048 (NOTE 1) | 2.5 |
| 3. | Disteardimonium hectorite | 0.2 |
| 4. | KMP-590 (NOTE 2) | 2 |
| 5. | Ethylhexyl methoxycinnamate | 7.5 |
| 6. | Octocrylene | 5 |
| 7. | Homosalate | 5 |
| 8. | Hexyl diethylaminohydroxybenzoylbenzoate | 2.5 |
| 9. | Bis-ethylhexyloxyphenol methoxyphenyl triazine | 2 |
| 10. | Ethylhexyl salicylate | 5 |
| 11. | Coco-caprylate/caprate | 12 |
| 12. | Ethyl isostearate | 10 |
| 13. | Hexyl laurate | 8 |
| 14. | Stearyl glycyrrhizin | 0.2 |
| 15. | BG | 3 |
| 16. | Ethanol | 6 |
| 17. | Sodium citrate | 0.2 |
| 18. | Sodium chloride | 1 |
| 19. | Purified water | Rest |
| Total | | 100 |

| | | |
|---|---|---|
| (NOTE 1) Cetyl PEG/PPG-10/1 dimethicone (:labeled name), manufactured by Shin-Etsu Chemical Co., Ltd. (NOTE 2) Polymethyl silsesquioxane (:labeled name), manufactured by Shin-Etsu Chemical Co., Ltd. | | |

The obtained W/O shaking sunscreen cream had good feel of use and coatability, and excellent storage stability.

### Example 35

### W/O Eye Cream

### <Preparation of Cosmetic>

A: The components 1 to 8 were mixed to be uniform;
B: The components 9 to 15 were mixed to be uniform; and
C: "B" was added into "A", and the mixture was emulsified to obtain an eye cream.

| Component | | mass% |
|---|---|---|
| 1. | KSG-840 (NOTE 1) | 3 |
| 2. | Composition of Example 20 (8.5%) | 3 |
| 3. | KSG-19 (NOTE 2) | 3 |
| 4. | KF-6105 (NOTE 3) | 1.5 |
| 5. | Simmondsia Chinensis (jojoba) seed oil | 6 |
| 6. | Limnanthes Alba (meadowfoam) seed oil | 12 |
| 7. | Shea fat | 1 |
| 8. | KSP-300 (NOTE 4) | 2 |
| 9. | Sodium hyaluronate | 0.1 |
| 10. | Erythritol | 5 |
| 11. | Methyl gluceth-10 | 3 |
| 12. | Phenoxyethanol | 0.3 |
| 13. | Pentylene glycol | 3 |
| 14. | Sodium chloride | 0.5 |
| 15. | Water | Rest |
| Total | | 100 |

| | | |
|---|---|---|
| (NOTE 1) Mixture of (lauryl dimethicone / polyglycerin-3) crosspolymer (:labeled name) 30% and squalane (:labeled name) 70%, manufactured by Shin-Etsu Chemical Co., Ltd. (NOTE 2) Mixture of (dimethicone / vinyl dimethicone) crosspolymer (labeled name) 15% and dimethicone (:labeled name) 85%, manufactured by Shin-Etsu Chemical Co., Ltd. (NOTE 3) Laurylpolyglyceryl-3 polydimethylsiloxy ethyl dimethicone (:labeled name), manufactured by Shin-Etsu Chemical Co., Ltd. (NOTE 4) (Diphenyl dimethicone / vinyl diphenyl dimethicone / silsesquioxane) crosspolymer (:labeled name), manufactured by Shin-Etsu Chemical Co., Ltd. | | |

The obtained W/O eye cream had good feel of use and coatability, and excellent storage stability.

### Example 36

### O/W Milky Lotion

### <Preparation of Cosmetic>

A: The components 1 to 6 were mixed to be uniform at 90°C;
B: The components 7 to 12 were mixed to be uniform at 85°C; and
C: "B" was added into "A" and emulsified, cooled, and the component 13 was added and mixed to be uniform to obtain a milky lotion.

| Component | | mass% |
|---|---|---|
| 1. | BG | 6 |
| 2. | PEG-20 glyceryl isostearate | 1.2 |
| 3. | Ethylhexyl palmitate | 4 |
| 4. | Composition of Example 23 (11%) | 2 |
| 5. | Polyquaternium-51 | 0.1 |
| 6. | Tocopherol | 0.05 |
| 7. | Diglycerin | 2 |
| 8. | Ethylhexylglycerin | 0.1 |
| 9. | Glycerin | 8 |
| 10. | Sorbitol | 3 |
| 11. | Alkyl-modified carbomer | 0.2 |
| 12. | Water | Rest |
| 13. | Potassium hydroxide (10% aqueous solution) | 0.5 |
| Total | | 100 |

The obtained O/W milky lotion had good feel of use and coatability, and excellent storage stability.

### Example 37

### Balm

### <Preparation of Cosmetic>

A: The components 1 to 11 were mixed to be uniform at 90°C, and the mixture was cooled and then a balm was obtained.

| Component | | mass% |
|---|---|---|
| 1. | Vaseline | 27 |
| 2. | Squalane | Rest |
| 3. | KSP-101 (NOTE 1) | 10 |
| 4. | KMP-590 (NOTE 2) | 6 |
| 5. | Composition of Example 19 (8.5%) | 5 |
| 6. | Composition of Example 23 (11%) | 3 |
| 7. | Dimethicone (6cs) | 7 |
| 8. | KF-56A (NOTE 3) | 4 |
| 9. | KP-561P (NOTE 4) | 2 |
| 10. | KF-6038 (NOTE 5) | 0.2 |
| 11. | Dextrin palmitate | 10 |
| Total | | 100 |

| | | |
|---|---|---|
| (NOTE 1) (Vinyl dimethicone / methicone silsesquioxane) crosspolymer (:labeled name), manufactured by Shin-Etsu Chemical Co., Ltd. (NOTE 2) Polymethyl silsesquioxane (:labeled name), manufactured by Shin-Etsu Chemical Co., Ltd. (NOTE 3) Diphenylsiloxy phenyl trimethicone (:labeled name), manufactured by Shin-Etsu Chemical Co., Ltd. (NOTE 4) (Acrylates / stearyl acrylate / dimethicone methacrylate) copolymer (:labeled name), manufactured by Shin-Etsu Chemical Co., Ltd. (NOTE 5) Lauryl PEG-9 polydimethylsiloxy ethyl dimethicone (:labeled name), manufactured by Shin-Etsu Chemical Co., Ltd. | | |

The obtained balm had good feel of use and coatability, and excellent storage stability.

### Example 38

### W/O Sunscreen Milk

### <Preparation of Cosmetic>

A: The components 1 to 7 were mixed to be uniform;
B: The components 10 to 16 were mixed to be uniform; and
C: "B" was added into "A" and emulsified, and the components 8 and 9 were added and mixed to be uniform to obtain a W/O sunscreen milk.

| Component | | mass% |
|---|---|---|
| 1. | KSG-210 (NOTE 1) | 3 |
| 2. | Composition of Example 24 (8.5%) | 2 |
| 3. | KF-6028 (NOTE 2) | 1 |
| 4. | Simmondsia Chinensis (jojoba) seed oil | 2 |
| 5. | Dimethylpolysiloxane (6cs) | 5 |
| 6. | KF-9021L (NOTE 3) | 2 |
| 7. | Isotridecyl isononanoate | 5 |
| 8. | SPD-T5L (NOTE 4) | 20 |
| 9. | SPD-Z5L (NOTE 5) | 28 |
| 10. | Dipropylene glycol | 2 |
| 11. | Sodium citrate | 0.2 |
| 12. | Sodium chloride | 1 |
| 13. | Arbutin | 2 |
| 14. | Dipotassium glycyrrhizin | 0.2 |
| 15. | Sodium pyrosulfite | 0.05 |
| 16. | Purified water | Rest |
| Total | | 100 |

| | | |
|---|---|---|
| (NOTE 1) Mixture of (dimethicone / (PEG-10/15)) crosspolymer (:labeled name) 25% and dimethicone 75%, manufactured by Shin-Etsu Chemical Co., Ltd. (NOTE 2) PEG-9 polydimethylsiloxy ethyl dimethicone (labeled name), manufactured by Shin-Etsu Chemical Co., Ltd. (NOTE 3) Trimethylsiloxylsilicic acid (:labeled name) (INCI: Trimethylsiloxysilicater), manufactured by Shin-Etsu Chemical Co., Ltd. (NOTE 4) Titanium oxide 40% dispersion, manufactured by Shin-Etsu Chemical Co., Ltd. (NOTE 5) Zinc oxide 60% dispersion, manufactured by Shin-Etsu Chemical Co., Ltd. | | |

The obtained W/O sunscreen milk had good feel of use and coatability, and excellent storage stability.

### Example 39

### O/W Sunscreen Cream

### <Preparation of Cosmetic>

A: The components 1 to 6 were heated to 85°C, and mixed to be uniform;
B: The components 7 to 15 were heated to 85°C, and mixed to be uniform; and
C: "B" was added into "A" and emulsified at 85°C, and then gradually cooled with stirring to obtain a sunscreen cream.

| Component | | mass% |
|---|---|---|
| 1. | Hydroxyethylcellulose | 0.1 |
| 2. | Ethanol | 10 |
| 3. | BG | 6 |
| 4. | Allantoin | 0.1 |
| 5. | SIMULGEL EG (NOTE 1) | 2.5 |
| 6. | Water | Rest |
| 7. | KF-56A (NOTE 2) | 3 |
| 8. | Composition of Example 20 (8.5%) | 1 |
| 9. | Cetanol | 2 |
| 10. | Ethylhexyl methoxycinnamate | 7.5 |
| 11. | Hexyl diethylaminohydroxybenzoylbenzoate | 2 |
| 12. | Ethylhexyl salicylate | 5 |
| 13. | Polyoxyethylene (60) hardened castor oil | 1 |
| 14. | KF-6011 (NOTE 3) | 0.5 |
| 15. | Tocopherol | 0.05 |
| Total | | 100 |

| | | |
|---|---|---|
| (NOTE 1) Copolymer composition of sodium acrylate and sodium acryloyldimethyltaurate, manufactured by SEPPIC (NOTE 2) Diphenylsiloxy phenyl trimethicone (:labeled name), manufactured by Shin-Etsu Chemical Co., Ltd. (NOTE 3) PEG-11 methyl ether dimethicone (:labeled name), manufactured by Shin-Etsu Chemical Co., Ltd. | | |

The obtained O/W sunscreen cream had good feel of use and coatability, and excellent storage stability.

### Example 40

### Sunscreen Gel

### <Preparation of Cosmetic>

A: The components 1 to 10 were mixed to be uniform to obtain a sunscreen gel.

| Component | | mass% |
|---|---|---|
| 1. | KSG-42A (NOTE 1) | 10 |
| 2. | Composition of Example 4 (8.5%) | 25 |
| 3. | KSG-042Z (NOTE 2) | Rest |
| 4. | KF-56A (NOTE 3) | 7.5 |
| 5. | Octocrylene | 2 |
| 6. | Homosalate | 10 |
| 7. | Ethylhexyl salicylate | 5 |
| 8. | Bis-ethylhexyloxyphenol methoxyphenyl triazine | 3 |
| 9. | KSP-441 (NOTE 4) | 10 |
| 10. | KMP-592 (NOTE 5) | 2 |
| Total | | 100 |

| | | |
|---|---|---|
| (NOTE 1) Mixture of (vinyl dimethicone / lauryl dimethicone) crosspolymer (labeled name) 20% and isododecane (:labeled name) 80%, manufactured by Shin-Etsu Chemical Co., Ltd. (NOTE 2) Mixture of (lauryl polydimethylsiloxy ethyl dimethicone / bisvinyl dimethicone) crosspolymer (:labeled name) 20% and isododecane (:labeled name) 80%, manufactured by Shin-Etsu Chemical Co., Ltd. (NOTE 3) Diphenylsiloxy phenyl trimethicone (:labeled name), manufactured by Shin-Etsu Chemical Co., Ltd. (NOTE 4) Polysilicone-22 (:labeled name), manufactured by Shin-Etsu Chemical Co., Ltd. (NOTE 5) (Methyl/phenyl) polysilsesquioxane (:labeled name), manufactured by Shin-Etsu Chemical Co., Ltd. | | |

The obtained sunscreen gel had good feel of use and coatability, and excellent storage stability and transparency.

### Example 41

### Non-Aqueous Sunscreen

### <Preparation of Cosmetic>

A: The components 1 to 6 were mixed to be uniformly dissolved;
B: The components 7 to 10 were mixed to be used; and
C: "A" and "B" were mixed to be uniform to obtain a non-aqueous sunscreen.

| Component | | mass% |
|---|---|---|
| 1. | Dimethicone (2cs) | Rest |
| 2. | Octocrylene | 5 |
| 3. | Hexyl diethylaminohydroxybenzoylbenzoate | 2 |
| 4. | Ethylhexyl salicylate | 5 |
| 5. | Stearyl glycyrrhetinate | 0.2 |
| 6. | BHT | 0.1 |
| 7. | KSG-380Z (NOTE 1) | 5 |
| 8. | Composition of Example 20 (8.5%) | 15 |
| 9. | SPD-Z5L (NOTE 2) | 58 |
| 10. | KSP-105 (NOTE 3) | 4 |
| Total | | 100 |

| | | |
|---|---|---|
| (NOTE 1) Mixture of (PEG-15 / lauryl polydimethylsiloxy ethyl dimethicone) crosspolymer (:labeled name) 30% and dimethicone, manufactured by Shin-Etsu Chemical Co., Ltd. (NOTE 2) Zinc oxide 60% dispersion, manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. | | |

The obtained non-aqueous sunscreen had good feel of use and coatability, and excellent storage stability.

### Example 42

### W/O Liquid Foundation

### <Preparation of Cosmetic>

A: The components 9 to 14 were dispersed with a triple-roller mill;
B: The components 1 to 8 were mixed to be uniform;
C: The components 15 to 18 were mixed to be uniform; and
D: "C" was added into "B" and emulsified, and "A" was added to obtain a W/O liquid foundation.

| Component | | mass% |
|---|---|---|
| 1. | KSF-210 (NOTE 1) | 3.5 |
| 2. | Composition of Example 20 (8.5%) | 5 |
| 3. | KF-6038 (NOTE 2) | 2.5 |
| 4. | Organic-modified clay mineral | 1 |
| 5. | Dimethicone (2cs) | 21 |
| 6. | Octocrylene | 5 |
| 7. | Polysilicone-15 | 2 |
| 8. | KSP-101 (NOTE 3) | 2 |
| 9. | Isononyl isononanoate | 3.7 |
| 10. | KP-578 (NOTE 4) | 0.3 |
| 11. | KTP-09W (NOTE 5) | 14 |
| 12. | KTP-09R (NOTE 5) | 0.2 |
| 13. | KTP-09Y (NOTE 5) | 0.5 |
| 14. | KTP-09B (NOTE 5) | 1 |
| 15. | BG | 5 |
| 16. | Sodium citrate | 0.2 |
| 17. | Sodium chloride | 1 |
| 18. | Water | Rest |
| Total | | 100 |

| | | |
|---|---|---|
| (NOTE 1) Mixture of (dimethicone / (PEG-10/15)) crosspolymer (:labeled name) 25% and dimethicone 75%, manufactured by Shin-Etsu Chemical Co., Ltd. (NOTE 2) Lauryl PEG-9 polydimethylsiloxy ethyl dimethicone (:labeled name), manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. (NOTE 4) (Acrylates / ethylhexyl acrylate / dimethicone methacrylate) copolymer (:labeled name), manufactured by Shin-Etsu Chemical Co., Ltd. (NOTE 5) KF-9909 treated colored inorganic pigment, W: white, R: red, Y: yellow, and B: black, manufactured by Shin-Etsu Chemical Co., Ltd. | | |

The obtained W/O liquid foundation had good feel of use and coatability, and excellent storage stability.

### Example 43

### W/O Solid Foundation

### <Preparation of Cosmetic>

A: The components 7 to 12 were dispersed with a triple-roller mill;
B: The components 1 to 6 were dissolved at 90°C, and "A" was added and mixed to be uniform;
C: The components 13 to 17 were mixed to be uniform; and
D: "C" was added into "B" and emulsified to obtain a W/O solid foundation.

| Component | | mass% |
|---|---|---|
| 1. | KSG-710 (NOTE 1) | 5 |
| 2. | Composition of Example 20 (8.5%) | 3 |
| 3. | KF-6038 (NOTE 2) | 2 |
| 4. | SPD-T7 (NOTE 3) | 10 |
| 5. | KF-56A (NOTE 4) | 7.35 |
| 6. | Ceresin | 5 |
| 7. | Isotridecyl isononanoate | 2 |
| 8. | Silicone-treated titanium oxide (NOTE 5) | 7 |
| 9. | Silicone-treated black iron oxide (NOTE 5) | 0.1 |
| 10. | Silicone-treated colcothar (NOTE 5) | 0.3 |
| 11. | Silicone-treated yellow iron oxide (NOTE 5) | 0.6 |
| 12. | KP-545 (NOTE 6) | 0.3 |
| 13. | BG | 7 |
| 14. | Glycerin | 5 |
| 15. | Sodium citrate | 0.2 |
| 16. | Sodium chloride | 1 |
| 17. | Water | Rest |
| Total | | 100 |

| | | |
|---|---|---|
| (NOTE 1) Mixture of (dimethicone / polyglycerin-3) crosspolymer (:labeled name) 25% and dimethicone 75%, manufactured by Shin-Etsu Chemical Co., Ltd. (NOTE 2) Lauryl PEG-9 polydimethylsiloxy ethyl dimethicone (labeled name), manufactured by Shin-Etsu Chemical Co., Ltd. (NOTE 3) Titanium oxide 45% dispersion, manufactured by Shin-Etsu Chemical Co., Ltd. (NOTE 4) Diphenylsiloxy phenyl trimethicone (:labeled name), manufactured by Shin-Etsu Chemical Co., Ltd. (NOTE 5) KF-9909 treated, manufactured by Shin-Etsu Chemical Co., Ltd. (NOTE 6) (Acrylates / dimethicone) copolymer (:labeled name), manufactured by Shin-Etsu Chemical Co., Ltd. | | |

The obtained W/O solid foundation had good feel of use and coatability, and excellent storage stability.

### Example 44

### Stick Concealer

### <Preparation of Cosmetic>

A: The components 1 to 7 were mixed to be uniformed at 95°C, and loaded into a stick container to obtain a stick concealer.

| Component | | mass% |
|---|---|---|
| 1. | Composition of Example 22 (8.5%) | 6 |
| 2. | KSP-411 (NOTE 1) | 10 |
| 3. | KMP-592 (NOTE 2) | 16 |
| 4. | Dimethicone (6cs) | 22 |
| 5. | Triethylhexanoin | 30 |
| 6. | Microcrystalline wax | 6 |
| 7. | Ceresin | 10 |
| Total | | 100 |

| | | |
|---|---|---|
| (NOTE 1) Polysilicone-1 crosspolymer (:labeled name), manufactured by Shin-Etsu Chemical Co., Ltd. (NOTE 2) (Methyl/phenyl) polysilsesquioxane (:labeled name), manufactured by Shin-Etsu Chemical Co., Ltd. | | |

The obtained stick concealer had good feel of use and coatability, and excellent storage stability.

### Example 45

### Lipstick

### <Preparation of Cosmetic>

A: The components 11 to 18 were dispersed with a roller;
B: The components 1 to 9 were mixed to be uniform at 95°C; and
C: "A" and the component 10 were added into "B", and cooled to obtain a lipstick.

| Component | | mass% |
|---|---|---|
| 1. | Composition of Example 21 (8.5%) | 5 |
| 2. | Polyethylene | 7 |
| 3. | Microcrystalline wax | 3 |
| 4. | KP-561P (NOTE 1) | 10 |
| 5. | Triethylhexanoin | 13 |
| 6. | Neopentyl glycol diethylhexanoate | 15 |
| 7. | Neopentyl glycol dicaprate | 7 |
| 8. | Hydrogenated polyisobutene | Rest |
| 9. | KF-54HV (NOTE 2) | 8 |
| 10. | Pearl pigment | 5 |
| 11. | Mica | 0.3 |
| 12. | Red 202 | 0.1 |
| 13. | Yellow 4 | 0.3 |
| 14. | KP-574 (NOTE 3) treated titanium oxide | 0.8 |
| 15. | KP-574 (NOTE 3) treated black iron oxide | 0.06 |
| 16. | Red 201 | 0.04 |
| 17. | KP-574 (NOTE 3) treated colcothar | 0.2 |
| 18. | Polyglyceryl-2 triisostearate | 1.2 |
| Total | | 100 |

| | | |
|---|---|---|
| (NOTE 1) (Acrylates / stearyl acrylate / dimethicone methacrylate) copolymer (:labeled name), manufactured by Shin-Etsu Chemical Co., Ltd. (NOTE 2) Diphenyl dimethicone (:labeled name), manufactured by Shin-Etsu Chemical Co., Ltd. (NOTE 3) (Acrylates / tridecyl acrylate / triethoxysilylpropyl methacrylate / dimethicone methacrylate) copolymer (:labeled name), manufactured by Shin-Etsu Chemical Co., Ltd. | | |

The obtained lipstick had good feel of use and coatability, and excellent storage stability.

### Example 46

### Mascara

### <Preparation of Cosmetic>

A: The components 1 to 7 were mixed to be uniform at 90°C; and
B: The components 8 to 13 were added into "A", and mixed to be uniform to obtain a mascara.

| Component | | mass% |
|---|---|---|
| 1. | Composition of Example 24 (8.5%) | 5 |
| 2. | TSPL-30-ID (NOTE 1) | 10 |
| 3. | Isododecane | Rest |
| 4. | Dextrin palmitate | 2 |
| 5. | Ceresin | 7 |
| 6. | Microcrystalline wax | 5 |
| 7. | Disteardimonium hectorite | 5 |
| 8. | Alkylsilane-treated black iron oxide (NOTE 2) | 5 |
| 9. | Alkylsilane-treated talc (NOTE 2) | 5 |
| 10. | KMP-590 (NOTE 3) | 5 |
| 11. | Silicone-branched polyether-modified silicone (NOTE 4) | 1.2 |
| 12. | Propylene carbonate | 1.6 |
| 13. | Phenoxyethanol | 0.2 |
| Total | | 100 |

| | | |
|---|---|---|
| (NOTE 1) Tri(methylsiloxy)silylcarbamoyl pullulan (:labeled name), manufactured by Shin-Etsu Chemical Co., Ltd. (NOTE 2) AES-3083 treated, manufactured by Shin-Etsu Chemical Co., Ltd. (NOTE 3) Polymethyl silsesquioxane (:labeled name), manufactured by Shin-Etsu Chemical Co., Ltd. (NOTE 4) PEG-9 polydimethylsiloxy ethyl dimethicone (:labeled name), manufactured by Shin-Etsu Chemical Co., Ltd. | | |

The obtained mascara had good feel of use and coatability, and excellent storage stability.

### Example 47

### Powder Foundation

### <Preparation of Cosmetic>

A: The components 1 to 5 were heated to 50°C, mixed to be uniform, and cooled to a room temperature;
B: The components 6 to 14 were mixed to be uniform; and
C: "A" was added into "B", and mixed to be uniform with a Henschel mixer. The obtained powder was passed through a mesh, and then punched onto a tray by using a mold to obtain a powder foundation.

| Component | | mass% |
|---|---|---|
| 1. | Ethylhexyl methoxycinnamate | 4 |
| 2. | KF-56A (NOTE 1) | 4.5 |
| 3. | Triethylhexanoin | 1.5 |
| 4. | KP-561P (NOTE 2) | 1 |
| 5. | Composition of Example 19 (8.5%) | 1 |
| 6. | Silicone-treated mica (NOTE 3) | 30 |
| 7. | Barium sulfate | 10 |
| 8. | KSP-300 (NOTE 4) | 5 |
| 9. | KSP-100 (NOTE 5) | 4 |
| 10. | Silicone-treated talc (NOTE 3) | Rest |
| 11. | Silicone-treated titanium oxide (NOTE 3) | 6 |
| 12. | Silicone-treated yellow iron oxide (NOTE 3) | 0.5 |
| 13. | Silicone-treated red iron oxide (NOTE 3) | 0.2 |
| 14. | Silicone-treated black iron oxide (NOTE 3) | 0.1 |
| Total | | 100 |

| | | |
|---|---|---|
| (NOTE 1) Diphenylsiloxy phenyl trimethicone (:labeled name), manufactured by Shin-Etsu Chemical Co., Ltd. (NOTE 2) (Acrylates / stearyl acrylate / dimethicone methacrylate) copolymer (:labeled name), manufactured by Shin-Etsu Chemical Co., Ltd. (NOTE 3) KF-9909 treated, manufactured by Shin-Etsu Chemical Co., Ltd. (NOTE 4) (Diphenyl dimethicone / vinyl diphenyl dimethicone / silsesquioxane) crosspolymer (:labeled name), manufactured by Shin-Etsu Chemical Co., Ltd. (NOTE 5) (Vinyl dimethicone / methicone silsesquioxane) crosspolymer (:labeled name), manufactured by Shin-Etsu Chemical Co., Ltd. | | |

The obtained powder foundation had good feel of use and coatability, and excellent storage stability.

### Example 48

### Hair Oil

### <Preparation of Cosmetic>

A: The components 1 to 7 were mixed to be uniform to obtain a hair oil.

| Component | mass% |
|---|---|
| 1. Composition of Example 22 (8.5%) | 3 |
| 2. KF-56A (NOTE 1) | 7 |
| 3. Diethylhexyl succinate | 10 |
| 4. X-21-5613 (NOTE 2) | 1.5 |
| 5. Tocopherol | 0.1 |
| 6. Perfume | 0.1 |
| 7. Light liquid isoparaffin | Rest |
| Total | 100 |

| | |
|---|---|
| (NOTE 1) Diphenylsiloxy phenyl dimethicone (:labeled name), manufactured by Shin-Etsu Chemical Co., Ltd. (NOTE 2) Dimethiconol (:labeled name), manufactured by Shin-Etsu Chemical Co., Ltd. | |

The obtained hair oil had good feel of use and coatability, and excellent storage stability.

### Example 49

### Hair Treatment

### <Preparation of Cosmetic>

A: The components 6 to 9 were heated to 70°C, and mixed to be uniform;
B: The components 1 to 5 were heated to 70°C, and mixed to be uniform; and
C: "B" was added into "A" and emulsified, gradually cooled, and then the components 10 and 11 were added to obtain a hair treatment.

| Component | | mass% |
|---|---|---|
| 1. Composition of Example 20 (8.5%) | | 1 |
| 2. Cetanol | | 2 |
| 3. Cetyl octanoate | | 3 |
| 4. Butyl paraoxybenzoate | | 0.1 |
| 5. KF-56A (NOTE 1) | | 1 |
| 6. Behentrimonium chloride | | 1 |
| 7. Propylene glycol | | 5 |
| 8. Hydroxyethylcellulose | | 0.1 |
| 9. Water | | Rest |
| 10. X-52-2328 (NOTE 2) | | 4 |
| 11. Perfume | Appropriate amount | |
| Total | | 100 |

| | | |
|---|---|---|
| (NOTE 1) Diphenylsiloxy phenyl dimethicone (:labeled name), manufactured by Shin-Etsu Chemical Co., Ltd. (NOTE 2) Aminopropyl dimethicone (:labeled name), manufactured by Shin-Etsu Chemical Co., Ltd. | | |

The obtained hair treatment had good feel of use and coatability, and excellent storage stability.

### Example 50

### W/O Sunscreen

### <Preparation of Cosmetic>

A: The components 1 to 9 were heated, and mixed to be uniform;
B: The components 11 to 14 were mixed to be uniform; and
C: "B" was added into "A" and emulsified, and then the component 10 was added and mixed to be uniform to obtain a W/O sunscreen.

| Component | mass% |
|---|---|
| 1. Composition of Example 20 (8.5%) | 1 |
| 2. KSG-240 (NOTE 1) | 3.5 |
| 3. KF-6048 (NOTE 2) | 2 |
| 4. Ethylhexyl methoxycinnamate | 7.5 |
| 5. Octocrylene | 2.5 |
| 6. Hexyl diethylaminohydroxybenzoylbenzoate | 3 |
| 7. Ethylhexyl salicylate | 3 |
| 8. Homosalate | 4 |
| 9. KF-56A (NOTE 3) | 2 |
| 10. SPD-T5 (NOTE 4) | 12 |
| 11. Ethanol | 6 |
| 12. Sodium citrate | 0.5 |
| 13. Sodium chloride | 0.5 |
| 14. Water | Rest |
| Total | 100 |

| | |
|---|---|
| (NOTE 1) Mixture of (dimethicone / (PEG-10/15)) crosspolymer (:labeled name) 20% and cyclopentasiloxane (:labeled name) 80%, manufactured by Shin-Etsu Chemical Co., Ltd. (NOTE 2) Cetyl PEG/PPG-10/1 dimethicone, manufactured by Shin-Etsu Chemical Co., Ltd. (NOTE 3) Diphenylsiloxy phenyl dimethicone (:labeled name), manufactured by Shin-Etsu Chemical Co., Ltd. (NOTE 4) Titanium oxide 40% dispersion, manufactured by Shin-Etsu Chemical Co., Ltd. | |

The obtained W/O sunscreen had good feel of use, coatability, and transparency, and excellent storage stability.

### Example 51

### W/O Liquid Foundation

### <Preparation of Cosmetic>

A: The components 9 to 15 were dispersed with a triple-roller mill;
B: The components 1 to 8 were mixed to be uniform;
C: The components 16 to 19 were mixed to be uniform; and
D: "C" was added into "B" and emulsified, and "A" was added to obtain a W/O liquid foundation.

| Component | mass% |
|---|---|
| 1. KSG-210 (NOTE 1) | 3.5 |
| 2. Composition of Example 14 (90%) | 5 |
| 3. KF-6038 (NOTE 2) | 2.5 |
| 4. Organic-modified cray mineral | 0.8 |
| 5. Dimethicone (2cs) | 21 |
| 6. Ethylhexyl methoxycinnamate | 7.5 |
| 7. Hexyl diethylaminohydroxybenzoylbenzoate | 2.5 |
| 8. KSP-101 (NOTE 3) | 2 |
| 9. TMF-1.5 (NOTE 4) | 6 |
| 10. KF-6105 (NOTE 5) | 0.5 |
| 11. KTP-09W (NOTE 6) | 10 |
| 12. KTP-09R (NOTE 6) | 0.2 |
| 13. KTP-09Y (NOTE 6) | 0.5 |
| 14. KTP-09B (NOTE 6) | 0.1 |
| 15. Alkylsilane-treated zinc oxide fine particle | 10 |
| 16. BG | 5 |
| 17. Sodium citrate | 0.2 |
| 18. Sodium chloride | 1 |
| 19. Water | Rest |
| Total | 100 |

| | |
|---|---|
| (NOTE 1) Mixture of (dimethicone / (PEG-10/15)) crosspolymer (:labeled name) 25% and dimethicone 75%, manufactured by Shin-Etsu Chemical Co., Ltd. (NOTE 2) Cetyl PEG/PPG-10/1 dimethicone (:labeled name), manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. (NOTE 4) Methyl trimethicone (:labeled name), manufactured by Shin-Etsu Chemical Co., Ltd. (NOTE 5) Lauryl polyglyceryl-3 polydimethylsiloxy ethyl dimethicone (:labeled name), manufactured by Shin-Etsu Chemical Co., Ltd. (NOTE 6) KF-9909 treated colored inorganic pigment, W: white, R: red, Y: yellow, and B: black, manufactured by Shin-Etsu Chemical Co., Ltd. | |

The obtained W/O liquid foundation had good feel of use and coatability, and excellent storage stability.

### Example 52

### W/O Liquid Foundation

### <Preparation of Cosmetic>

A: The components 9 to 15 were dispersed with a triple-roller mill;
B: The components 1 to 8 were mixed to be uniform;
C: The components 16 to 19 were mixed to be uniform; and
D: "C" was added into "B" and emulsified, and "A" was added to obtain a W/O liquid foundation.

| Component | mass% |
|---|---|
| 1. KSG-210 (NOTE 1) | 3.5 |
| 2. Composition of Example 20 (8.5%) | 5 |
| 3. KF-6028 (NOTE 2) | 2.5 |
| 4. Organic-modified cray mineral | 0.8 |
| 5. Dimethicone (2cs) | 21 |
| 6. Ethylhexyl palmitate | 7 |
| 7. Caprylic/capric triglyceride | 5 |
| 8. KSP-101 (NOTE 3) | 2 |
| 9. KF-56A (NOTE 4) | 7 |
| 10. KF-6106 (NOTE 5) | 1 |
| 11. KTP-09W (NOTE 6) | 10 |
| 12. KTP-09R (NOTE 6) | 0.2 |
| 13. KTP-09Y (NOTE 6) | 0.5 |
| 14. KTP-09B (NOTE 6) | 0.1 |
| 15. Metal soap-treated titanium oxide fine particle | 10 |
| 16. BG | 5 |
| 17. Sodium citrate | 0.2 |
| 18. Sodium chloride | 1 |
| 19. Water | Rest |
| Total | 100 |

| | |
|---|---|
| (NOTE 1) Mixture of (dimethicone / (PEG-10/15)) crosspolymer (:labeled name) 25% and dimethicone 75%, manufactured by Shin-Etsu Chemical Co., Ltd. (NOTE 2) PEG-9 polydimethylsiloxy ethyl dimethicone (:labeled name), manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. (NOTE 4) Diphenylsiloxy phenyl dimethicone (:labeled name), manufactured by Shin-Etsu Chemical Co., Ltd. (NOTE 5) Lauryl polyglyceryl-3 polydimethylsiloxy ethyl dimethicone (:labeled name), manufactured by Shin-Etsu Chemical Co., Ltd. (NOTE 6) KF-9909 treated colored inorganic pigment, W: white, R: red, Y: yellow, and B: black, manufactured by Shin-Etsu Chemical Co., Ltd. | |

The obtained W/O liquid foundation had good feel of use and coatability, and excellent storage stability.

### Example 53

### Sun-Care Stick

### <Preparation of Cosmetic>

A: The components 1 to 14 were mixed to be uniform at 110°C, and loaded into a stick container to obtain a sun-care stick.

| Component | mass% |
|---|---|
| 1. Composition of Example 20 (8.5%) | 6 |
| 2. KF-56A (NOTE 1) | Rest |
| 3. KMP-592 (NOTE 2) | 0.5 |
| 4. Diisopropyl sebacate | 10 |
| 5. Ethylhexyl methoxycinnamate | 7.5 |
| 6. Octocrylene | 2.5 |
| 7. Hexyl diethylaminohydroxybenzoylbenzoate | 5 |
| 8. Ethylhexyl salicylate | 5 |
| 9. Bis-ethylhexyloxyphenol methoxyphenyl triazine | 3 |
| 10. Polysilicone-15 | 2 |
| 11. Homosalate | 10 |
| 12. Dibutyl lauroyl glutamide | 3.5 |
| 13. Octyldodecanol | 10 |
| 14. Mineral oil | 2 |
| Total | 100 |

| | |
|---|---|
| (NOTE 1) Diphenylsiloxy phenyl dimethicone (:labeled name), manufactured by Shin-Etsu Chemical Co., Ltd. (NOTE 2) (Methyl/phenyl) polysilsesquioxane (:labeled name), manufactured by Shin-Etsu Chemical Co., Ltd. | |

The obtained sun-care stick foundation had good feel of use, coatability, and transparency, and excellent storage stability.

### Example 54

### W/O Cosmetic Stick

### <Preparation of Cosmetic>

A: The components 1 to 6 were mixed to be uniform at 90°C;
B: The components 7 to 12 were mixed to be uniform at 85°C; and
C: "B" was added into "A" and emulsified, and loaded into a stick container to obtain a W/O cosmetic stick.

| Component | mass% |
|---|---|
| 1. KSG-830 (NOTE 1) | 5 |
| 2. Composition of Example 23 (11%) | 5 |
| 3. KF-6105 (NOTE 2) | 0.5 |
| 4. Limnanthes Alba (meadowfoam) seed oil | 2 |
| 5. KF-56A (NOTE 3) | 8 |
| 6. Ceresin | 10 |
| 7. BG | 7 |
| 8. PCA-Na | 2 |
| 9. Diglycerin | 3 |
| 10. Sodium citrate | 0.2 |
| 11. Magnesium sulfate | 1 |
| 12. Water | Rest |
| Total | 100 |

| | |
|---|---|
| (NOTE 1) Mixture of (lauryl dimethicone / polyglycerin-3) crosspolymer (:labeled name) 20% and triethylhexanoin (:labeled name) 80%, manufactured by Shin-Etsu Chemical Co., Ltd. (NOTE 2) Lauryl PEG-9 polydimethylsiloxy ethyl dimethicone (labeled name), manufactured by Shin-Etsu Chemical Co., Ltd. (NOTE 3) Diphenylsiloxy phenyl trimethicone (:labeled name), manufactured by Shin-Etsu Chemical Co., Ltd. | |

The obtained W/O cosmetic stick had good feel of use and coatability, and excellent storage stability.

### Example 55

### W/O Stick Foundation

### <Preparation of Cosmetic>

A: The components 12 to 17 were dispersed with a triple-roller mill;
B: The components 1 to 11 were dissolved at 90°C, and "A" was added and mixed to be uniform;
C: The components 18 to 23 were mixed to be uniform; and
D: "C" was added into "B" and emulsified, and loaded into a stick container to obtain a W/O stick foundation.

| Component | mass% |
|---|---|
| 1. KSG-710 (NOTE 1) | 5 |
| 2. Composition of Example 19 (8.5%) | 3 |
| 3. KF-6038 (NOTE 2) | 2 |
| 4. KF-995 (NOTE 3) | 10 |
| 5. Stearoyl inulin | 3 |
| 6. Synthetic wax | 7 |
| 7. t-Butylmethoxydibenzoylmethane | 2 |
| 8. Ethylhexyl salicylate | 4 |
| 9. Bis-ethylhexyloxyphenol methoxyphenyl triazine | 2 |
| 10. Polysilicone-15 | 2 |
| 11. Homosalate | 5 |
| 12. Isotridecyl isononanoate | 2 |
| 13. KP-578 (NOTE 4) | 0.3 |
| 14. Silicone-treated titanium oxide (NOTE 5) | 7 |
| 15. Silicone-treated black iron oxide (NOTE 5) | 0.1 |
| 16. Silicone-treated colcothar (NOTE 5) | 0.3 |
| 17. Silicone-treated yellow iron oxide (NOTE 5) | 0.6 |
| 18. BG | 7 |
| 19. Glycerin | 5 |
| 20. Sodium citrate | 0.2 |
| 21. Sodium chloride | 1 |
| 22. Disodium edetate | 0.1 |
| 23. Water | Rest |
| Total | 100 |

| | |
|---|---|
| (NOTE 1) Mixture of (dimethicone / polyglycerin-3) crosspolymer (:labeled name) 25% and dimethicone 75%, manufactured by Shin-Etsu Chemical Co., Ltd. (NOTE 2) Lauryl PEG-9 polydimethylsiloxy ethyl dimethicone (labeled name), manufactured by Shin-Etsu Chemical Co., Ltd. (NOTE 3) Cyclopentasiloxane (:labeled name), manufactured by Shin-Etsu Chemical Co., Ltd. (NOTE 4) (Acrylates / ethylhexyl acrylate / dimethicone methacrylate) copolymer (:labeled name), manufactured by Shin-Etsu Chemical Co., Ltd. (NOTE 5) KF-9901 treated, manufactured by Shin-Etsu Chemical Co., Ltd. | |

The obtained W/O stick foundation had good feel of use and coatability, and excellent storage stability.

### Example 56

### Mousse Foundation

### <Preparation of Cosmetic>

A: The components 9 to 16 were dispersed with a triple-roller mill;
B: The components 1 to 8 were mixed to be uniform; and
C: "A" was added into "B", and mixed to be uniform to obtain a mousse foundation.

| Component | | mass% |
|---|---|---|
| 1. | KSG-16 (NOTE 1) | 20 |
| 2. | Composition of Example 8 (14%) | 12 |
| 3. | KF-7312T (NOTE 2) | 8 |
| 4. | Neopentyl glycol diethylhexanoate | 4 |
| 5. | Dimethicone (2cs) | Rest |
| 6. | Simmondsia Chinensis (jojoba) oil | 0.5 |
| 7. | KSP-105 (NOTE 3) | 2 |
| 8. | Polymethyl methacrylate | 6 |
| 9. | Dimethicone (6cs) | 10 |
| 10. | Silicone-treated titanium oxide (NOTE 4) | 5 |
| 11. | Metal soap-treated titanium oxide fine particle | 10 |
| 12. | Silicone-treated red iron oxide (NOTE 4) | 0.2 |
| 13. | Silicone-treated yellow iron oxide (NOTE 4) | 0.5 |
| 14. | Silicone-treated black iron oxide (NOTE 4) | 0.1 |
| 15. | Silicone-treated talc (NOTE 4) | 3 |
| 16. | Silicone-treated black mica (NOTE 4) | 5 |
| Total | | 100 |

| | | |
|---|---|---|
| (NOTE 1) Mixture of (dimethicone / vinyl dimethicone) crosspolymer (labeled name) 25% and dimethicone (:labeled name) 75%, manufactured by Shin-Etsu Chemical Co., Ltd. (NOTE 2) Trimethylsiloxysilicic acid (:labeled name (INCI: Trimethylsiloxysilicater)), manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. (NOTE 4) KF-9908 treated, manufactured by Shin-Etsu Chemical Co., Ltd. | | |

The obtained mousse foundation had good feel of use and coatability, and excellent storage stability.

### Example 57

### Mousse Foundation

### <Preparation of Cosmetic>

A: The components 12 to 19 were dispersed with a tripleroller mill;
B: The components 1 to 11 were mixed to be uniform; and
C: "A" was added into "B", and mixed to be uniform to obtain a mousse foundation.

| Component | | mass% |
|---|---|---|
| 1. | KSG-18A (NOTE 1) | 8 |
| 2. | Composition of Example 24 (8.5%) | 25 |
| 3. | KF-7312J (NOTE 2) | 8 |
| 4. | Cyclopentasiloxane | Rest |
| 5. | Dimethylsilylated silica | 0.5 |
| 6. | Ethylhexyl methoxycinnamate | 7 |
| 7. | Hexyl diethylaminohydroxybenzoylbenzoate | 2 |
| 8. | Bis-ethylhexyloxyphenol methoxyphenyl triazine | 1 |
| 9. | Homosalate | 5 |
| 10. | KSP-105 (NOTE 3) | 2 |
| 11. | Polymethyl methacrylate | 6 |
| 12. | KF-56A (NOTE 4) | 10 |
| 13. | Alkylsilane-treated titanium oxide (NOTE 5) | 5 |
| 14. | Metal soap-treated titanium oxide fine particle | 8 |
| 15. | Alkylsilane-treated red iron oxide (NOTE 5) | 0.2 |
| 16. | Alkylsilane-treated yellow iron oxide (NOTE 5) | 0.5 |
| 17. | Alkylsilane-treated black iron oxide (NOTE 5) | 0.1 |
| 18. | Alkylsilane-treated talc (NOTE 5) | 3 |
| 19. | Alkylsilane-treated black mica (NOTE 5) | 4 |
| Total | | 100 |

| | | |
|---|---|---|
| (NOTE 1) Mixture of (dimethicone / phenyl vinyl dimethicone) crosspolymer (labeled name) 15% and dimethicone (:labeled name) 85%, manufactured by Shin-Etsu Chemical Co., Ltd. (NOTE 2) Trimethylsiloxysilicic acid (:labeled name (INCI: Trimethylsiloxysilicater)), manufactured by Shin-Etsu Chemical Co., Ltd. (NOTE 3) (Vinyl dimethicone / methicone silsesquioxane) crosspolymer (:labeled name), manufactured by Shin-Etsu Chemical Co., Ltd. (NOTE 4) Diphenylsiloxy phenyl trimethicone (:labeled name), manufactured by Shin-Etsu Chemical Co., Ltd. (NOTE 5) AES-3083 treated, manufactured by Shin-Etsu Chemical Co., Ltd. | | |

The obtained mousse foundation had good feel of use and coatability, and excellent storage stability.

### Example 58

### Gel Eye Color

### <Preparation of Cosmetic>

A: The components 1 to 3 were mixed to be uniform at 80°C;
B: "A" and the components 4 to 5 were mixed to be uniform at 60°C; and
C: "B" and the components 6 to 11 were mixed to be uniform to obtain a gel eye color.

| Component | | mass% |
|---|---|---|
| 1. | Isotridecyl isononanoate | 24 |
| 2. | Squalane | Rest |
| 3. | Dextrin palmitate | 10 |
| 4. | Composition of Example 21 (8.5%) | 12 |
| 5. | Dimethylsilylated silica | 0.1 |
| 6. | KSP-100 (NOTE 1) | 8 |
| 7. | KMP-590 (NOTE 2) | 2 |
| 8. | Barium sulfate | 5 |
| 9. | Silicone-treated synthetic mica (NOTE 3) | 13 |
| 10. | Glass powder | 7 |
| 11. | (PET/Al) laminate | 4.5 |
| Total | | 100 |

| | | |
|---|---|---|
| (NOTE 1) (Vinyl dimethicone / methicone silsesquioxane) crosspolymer (:labeled name), manufactured by Shin-Etsu Chemical Co., Ltd. (NOTE 2) Polymethyl silsesquioxane (:labeled name), manufactured by Shin-Etsu Chemical Co., Ltd. (NOTE 3) KP-574 treated, manufactured by Shin-Etsu Chemical Co., Ltd. | | |

The obtained gel eye color had good feel of use and coatability, and excellent storage stability.

### Example 59

### Eye Color

### <Preparation of Cosmetic>

A: The components 1 to 5 were mixed to be uniform;
B: The components 6 to 15 were mixed to be uniform; and
C: "A" was added into "B", and mixed to be uniform with a Henschel mixer. The obtained powder was passed through a mesh, and then punched onto a tray by using a mold to obtain an eye color.

| Component | | mass% |
|---|---|---|
| 1. | Diisostearyl malate | 3 |
| 2. | KF-56A (NOTE 1) | 2 |
| 3. | Triethylhexanoin | 1.5 |
| 4. | Composition of Example 22 (8.5%) | 3 |
| 5. | Vaseline | 2 |
| 6. | KMP-590 (NOTE 2) | 2 |
| 7. | Silicone-treated mica (NOTE 3) | Rest |
| 8. | Silicone-treated talc (NOTE 3) | 25 |
| 9. | Barium sulfate | 3 |
| 10. | Synthetic fluorphlogopite | 10 |
| 11. | Boron nitride | 2 |
| 12. | KSP-300 (NOTE 4) | 4 |
| 13. | Zinc myristate | 0.5 |
| 14. | Sorbitan sesquiisostearate | 0.2 |
| 15. | Glass-based pearl pigment | 12 |
| Total | | 100 |

| | | |
|---|---|---|
| (NOTE 1) Diphenylsiloxy phenyl trimethicone (:labeled name), manufactured by Shin-Etsu Chemical Co., Ltd. (NOTE 2) Polymethyl silsesquioxane (:labeled name), manufactured by Shin-Etsu Chemical Co., Ltd. (NOTE 3) KF-9909 treated, manufactured by Shin-Etsu Chemical Co., Ltd. (NOTE 4) (Diphenyl dimethicone / vinyl diphenyl dimethicone / silsesquioxane) crosspolymer (:labeled name), manufactured by Shin-Etsu Chemical Co., Ltd. | | |

The obtained eye color had good feel of use and coatability, and excellent storage stability.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A cosmetic, comprising the following component (A),
the component (A): a hydrosilylated reaction product of the following (A1) and (A2), the hydrosilylated reaction product being an aromatic-group-modified crosslinked organosiloxane, and a ratio between a total number of moles of aryl groups and aralkyl groups and a number of moles of silicon atoms is 0.34 or more,
(A1) an organohydrogen (poly)siloxane represented by the following general formula (1) and having two or more silicon-atom-bonded hydrogen atoms in one molecule,
Mₐ₁M'ₐ₂D_{b1}D'_{b2}T_{c1}T'_{c2}Q_{d} (1)
wherein
M represents a siloxane unit represented by R¹₃SiO_{1/2},
M' represents a siloxane unit represented by R¹₂HSiO_{1/2},
D represents a siloxane unit represented by R¹₂SiO_{2/2},
D' represents a siloxane unit represented by R¹HSiO_{2/2},
T represents a siloxane unit represented by R¹SiO_{3/2},
T' represents a siloxane unit represented by HSiO_{3/2}, and
Q represents a siloxane unit represented by SiO_{4/2},
wherein R¹ independently represents a group selected from the group consisting of an alkyl group, aryl group, and aralkyl group having 1 to 30 carbon atoms; a1, a2, b1, b2, c1, c2, and d respectively represent integers of 0≤a1, 0≤a2, 0≤b1, 0≤b2, 0≤c1, 0≤c2, 0≤d, 2≤a2+b2+c2≤50, and 2≤a1+a2+b1+b2+c1+c2+d≤100,000, and
(A2) a linear organo(poly)siloxane represented by the following general formula (2) and having two or more alkenyl groups having 2 to 8 carbon atoms in one molecule,
Mₑ₁M"ₑ₂D_{f1}D"_{f2} (2)
wherein M and D are same as above,
M" represents a siloxane unit represented by R¹₂R²SiO_{1/2}, and
D" represents a siloxane unit represented by R¹R²SiO_{2/2},
wherein R¹ is same as above; R² represents the alkenyl group having 2 to 8 carbon atoms; e1, e2, f1,
and f2 respectively represent integers of 0≤e1≤2, 0≤e2≤2, 0≤f1, 0≤f2, e1+e2=2, 2≤e2+f2≤50, and 2≤e1+e2+f1+f2≤100,000.

2. The cosmetic according to claim 1, wherein in the general formula (2), f2=0.

3. The cosmetic according to claim 1 or 2, wherein the component (A) is a hydrosilylated reaction product of the following additional (A3),
(A3) a linear organohydrogen (poly)siloxane represented by the following general formula (3) and having one silicon-atom-bonded hydrogen atom in one molecule,
M_{g1}M'_{g2}Dₕ (3)
wherein M, M', and D are same as above; g1, g2, and h respectively represent integers of g1=g2=1 and 0≤h≤100.

4. The cosmetic according to any one of claims 1 to 3, wherein the component (A) is a hydrosilylated reaction product of the following additional (A4),
(A4) a linear organo(poly)siloxane represented by the following general formula (4) and having one alkenyl group having 2 to 8 carbon atoms in one molecule,
Mᵢ₁M"ᵢ₂Dⱼ (4)
wherein M, M", and D are same as above; i1, i2, and j respectively represent integers of i1=i2=1 and 0≤j≤100.

5. The cosmetic according to any one of claims 1 to 4, further comprising an oil agent (B) being liquid at 25°C in addition to the component (A).

6. The cosmetic according to claim 5, wherein the component (B) is one or more selected from a modified silicone oil, a hydrocarbon oil, a fluorine-based oil, an ester oil, a natural animal or vegetable oil, and a semisynthetic oil.

7. The cosmetic according to claim 5 or 6, wherein the component (A) and a part of the component (B) are mixed in advance.

8. The cosmetic according to any one of claims 1 to 7, further comprising an ultraviolet-ray absorbent as a component (C).
